# EUROPEAN PATENT SPECIFICATION

(11) **EP 4 171 559 B1**
(45) Date of publication and mention of the grant of the patent: **10.09.2025**
(21) Application number: 21733160.2
(22) Date of filing: 24.06.2021
(51) Int. Cl.: A61K 31/4545, A61K 45/06, A61P 3/00, A61P 25/00, A61P 43/00

(54) **ARIMOCLOMOL FOR TREATING GAUCHER DISEASE**
ARIMOCLOMOL ZUR BEHANDLUNG VON MORBUS GAUCHER
ARIMOCLOMOL POUR LE TRAITEMENT DE LA MALADIE DE GAUCHER

(30) Priority: 24.06.2020 EP 20182067
(43) Date of publication of application: 03.05.2023
(73) Proprietor: Zevra Denmark A/S, 2000 Frederiksberg (DK)
(72) Inventor: JENSEN, Thomas, Kirkegaard, 2610 Rødovre (DK); HINSBY, Anders, Mørkeberg, 2900 Hellerup (DK); FOG-TØNNESEN, Cathrine, Kolster, 2830 Virum (DK); INGEMANN, Linda, 2870 Dyssegård (DK); Í DALI, Christine, 1316 Copenhagen K (DK)
(74) Representative: Høiberg P/S
(86) International application number: PCT/EP2021/067366
(87) International publication number: WO 2021/260120

(56) References cited:
- WO-A1-2017/186919
- INGEMANN LINDA ET AL: "Rescue of NPC1 protein and effect on biomarkers by arimoclomol treatment in Niemann-Pick disease type C", MOLECULAR GENETICS AND METABOLISM, ACADEMIC PRESS, AMSTERDAM, NL, vol. 129, no. 2, 31 January 2020 (2020-01-31), XP086021455, ISSN: 1096-7192, DOI: 10.1016/J.YMGME.2019.11.190
- FOG CATHRINE K. ET AL: "The heat shock protein amplifier arimoclomol improves refolding, maturation and lysosomal activity of glucocerebrosidase", EBIOMEDICINE, vol. 38, December 2018 (2018-12-01), NL, pages 142 - 153, XP055838833, ISSN: 2352-3964, DOI: 10.1016/j.ebiom.2018.11.037
- ANONYMOUS: "Study of Arimoclomol in Patients Diagnosed With Gaucher Disease Type 1 or 3", CLINICALTRIALS.GOV, 4 September 2019 (2019-09-04), XP055838721, Retrieved from the Internet <URL:https://www.clinicaltrials.gov/ct2/show/NCT03746587?term=arimoclomol&cond=Gaucher+Disease&draw=2&rank=1> [retrieved on 20210907]
- AERTS JOHANNES M F G ET AL: "Glycosphingolipids and lysosomal storage disorders as illustrated by gaucher disease", CURRENT OPINION IN CHEMICAL BIOLOGY, CURRENT BIOLOGY LTD, LONDON, GB, vol. 53, 26 November 2019 (2019-11-26), pages 204 - 215, XP085960592, ISSN: 1367-5931, [retrieved on 20191126], DOI: 10.1016/J.CBPA.2019.10.006

## Description

### Technical field

The present disclosure relates to an active pharmaceutical ingredient selected from N-[2-hydroxy-3-(1-piperidinyl)-propoxy]-pyridine-1-oxide-3-carboximidoyl chloride, its stereoisomers and the acid addition salts thereof; specifically arimoclomol, for use in improved methods of treating Gaucher disease (GD) type 1 (GD1) or type 3 (GD3).

### Background

Gaucher disease is an autosomal recessive lysosomal storage disorder (LSD), which is characterised by a deficiency of glucocerebrosidase (GBA) enzyme. The enzyme deficiency is caused by mutations in the gene GBA1 that lead to accumulation of glucosylceramide (GlcCer) and its deacylated form glucosylsphingosine (GlcSph). Glucosylsphingosine accumulates in the brain and is assumed to be responsible for the neurological manifestations of GD.

The disease is characterised by lysosomal dysfunction, cell stress, and death caused by significantly reduced enzyme activity. This is in most cases due to the L444P substitution, where homozygosity is strongly associated with GD Type 3 (GD3). The L444P genotype has previously been demonstrated to respond to a number of heat shock protein (HSP)-inducing strategies.

Gaucher disease has traditionally been classified into 3 subtypes based on the absence or presence of primary central nervous system (CNS) involvement: GD Type 1 (GD1) with no neurological symptoms, GD Type 2 (GD2) with acute early neurological disease, and GD3 with more slow and chronic neuropathic disease.

The clinical onset of GD1 can occur at any age, but if symptoms begin in childhood the phenotype is typically more severe, with a more rapid rate of progression. The most frequent signs of disease are splenomegaly with anaemia and thrombocytopenia (mostly due to hypersplenism), hepatomegaly, and bone disease. The signs and symptoms related to hepatosplenomegaly may range from none to abdominal distension, discomfort, or pain. Thrombocytopenia results in increased tendency for bleeding and bruising, and anaemia can cause fatigue and failure to thrive with low weight and growth. Bone marrow infiltration by Gaucher cells leads to osteopenia, osteonecrosis, osteosclerosis, and sometimes acute episodes of excruciating pain (bone crises), chronic bone pain, and pathological fracture which substantially impacts the patient's quality of life.

Gaucher disease Type 3 is characterised as a milder chronic neurological disease compared to GD2 (acute form). The symptoms begin later in childhood, with or without the visceral and bone marrow involvement as seen in GD1. The earliest CNS involvement can be assessed by a detailed ophthalmologic examination revealing horizontal saccade (fast eye movement) initiation abnormalities, strabismus, and bulbar palsy or paresis. Neurologic progression is marked by severe hypertonia, rigid arching (opisthotonus), ataxia, swallowing impairment, and seizures. Visceral disease in GD3 is often more severe than in GD1. In both GD1 and GD3, clinical scoring scales have been developed and validated in order to be able to assess disease severity and possible clinical effect of treatment.

Two types of treatment are currently available for patients with GD: enzyme replacement therapy (ERT) using recombinant GBA enzyme, and substrate reduction therapy (SRT), which works by inhibiting GlcCer synthase, thereby reducing the generation of substrate. Both types of treatment improve the peripheral features of the disease, but not the neurologic symptoms, and hence are ineffective for treatment of neuropathic GD.

Accumulation of GlcCer and GlcSph inside lysosomes occurs primarily in mononuclear-macrophages and results in engorged macrophages called "Gaucher cells". In brain tissue from GD patients with neurological symptoms, neuronal loss, and crumpled, shrunken-atrophic neurons in the basal ganglia and nuclei of the midbrain, pons and medulla, cerebellum, dentate nucleus, and hypothalamus have been described.

Due to activated macrophages, there is a chronic stimulation of the immune system. Although there is a high variation among patients, increased levels of several interleukins ([IL]-1*α*, IL-1*β*, IL-1Ra, sIL-2R, IL-6, IL-8, IL-10, IL-18), tumour necrosis factor (TNF)-*α*, transforming growth factor-β, macrophage-colony stimulating factor, macrophage inflammatory protein-1 (MIP-1), and chemokine (C-C motif) ligand 18 have been reported. The most important biomarker, chitotriosidase, is an enzyme massively produced by the macrophages and neutrophils. Histochemistry of bone marrow aspirates and sections of spleens reveals these storage cells.

The HSR is a key homeostatic system, which is induced under conditions of metabolic stress (e.g., protein misfolding and aggregation, nutrient deprivation, oxidative or thermal stress). Its main components are the HSPs (in particular HSP70), which have significant cytoprotective properties. Heat shock protein 70 acts as molecular chaperone, assisting in the folding of newly synthesised damaged proteins, preventing protein aggregation, and targeting severely damaged proteins for degradation. Notably, the cytoprotective actions of HSP70 also include protection against lysosomal dysfunction, cell death, and demyelination in mouse studies. Arimoclomol is a safe and well-tolerated co-inducer of HSP70 expression that acts through the activation of heat shock factor-1, the major regulator of HSP gene transcription. It is available for oral administration and show ability to cross the blood-brain barrier.

WO 2017/186919 discloses arimoclomol for treating GBA-deficiencies other than Gaucher Disease, including GBA-deficient Parkinson's Disease (PD).

Fog et al., EBioMedicine 38 (2018), 142-153, discloses *in vitro* testing of arimoclomol in primary cells from Gaucher disease patients to demonstrate HSP induction and folding of mutated GBA in these cells.

Anonymous "Study of Arimoclomol in Patients Diagnosed with Gaucher Disease type 1 or 3", ClinicalTrials.gov, 4 September 2019 (2019-09-04), XP055838712, is a clinical study protocol for arimoclomol in GD.

Aerts et al., Current Opinion In Chemical Biology, 2019, 53:204-215, discloses clinical manifestations of Gaucher disease.

### Summary

The invention is defined by the claims. Any subject matter falling outside the scope of the claims is provided for information purposes only.

A double-blind, randomized, placebo-controlled, phase 2 dose-finding trial in Gaucher disease (GD) type 1 and 3 patients naïve to enzyme and/or substrate replacement therapy, was conducted at seven sites in India. A total of 39 patients were randomized 1:1:1:1 to receive placebo or 100mg, 200mg, or 400mg arimoclomol citrate (weight-adjusted) three times per day. The objective of the phase 2 study was to evaluate the response of the three dose levels of arimoclomol on various clinical and disease-specific biomarkers over a 6-month treatment period. Overall, 37 patients were included in the analysis set (2 patients excluded due to negative confirmatory GD genotype), of which 21 were type 1 GD and 16 were type 3 GD patients.

The data presented herewith show a dose-dependent effect of arimoclomol on certain disease-relevant clinical secondary endpoints, including liver and spleen size. Arimoclomol also demonstrated a relative reduction in serum chitotriosidase activity, and patients anemic at baseline showed a time-dependent increase in haemoglobin in the highest dose group.

The present disclosure provides an active pharmaceutical ingredient selected from *N-*[2-hydroxy-3-(1-piperidinyl)-propoxy]-pyridine-1-oxide-3-carboximidoyl chloride, its stereoisomers and the acid addition salts thereof, for use in a method of treating Gaucher disease (GD) type 1 (GD1) or GD type 3 (GD3); such as wherein said active pharmaceutical ingredient is administered at 100 mg t.i.d, such as 200 mg t.i.d., such as 400 mg t.i.d. (corresponding to 300 mg/day, such as 600 mg/day, such as 1200 mg/day), or in corresponding weigh-adjusted dosages.

The present disclosure also provides an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of reducing liver size / treating hepatomegaly and reducing spleen size / treating splenomegaly, as well as treating hepatosplenomegaly, in a patient with Gaucher disease type 1 (GD1) or type 3 (GD3).

**The** present disclosure further provides an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of treating splenomegaly or hepatosplenomegaly with anaemia and thrombocytopenia; such as treating anaemia; such as increasing haemoglobin, in a patient with Gaucher disease type 1 (GD1) or type 3 (GD3).

The present disclosure further provides an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of reducing serum chitotriosidase activity and/or increasing glycosylsphingosine (lyso-Gb1) in a patient with Gaucher disease type 1 (GD1) or type 3 (GD3).

### Description of Drawings

**Figure 1** shows the estimated plasma exposure of arimoclomol for the patients in the 3 arimoclomol dose groups. Plasma exposure is estimated using population pharmacokinetic modelling. The AUC0-8 (area under the curve from time 0 to 8 hours) is depicted for each patient of the 3 dose groups.
**Figure 2** shows the relative change in chitotriosidase activity from baseline versus placebo for each of the 3 arimoclomol dose groups.
**Figure 3** shows the relative change in liver size from baseline versus placebo for each of the 3 arimoclomol dose groups.
**Figure 4** shows the relative change in spleen size from baseline versus placebo for each of the 3 arimoclomol dose groups.
**Figure 5** shows the mean haemoglobin concentration in g/L over time of patient anaemic at baseline in the highest arimoclomol dose group (1200 mg/day).
**Figure 6** shows the change from baseline of plasma glycosylsphingosine (lyso-Gb1) measured after 6 months.

### Detailed description

### Definitions

The term "pharmaceutically acceptable derivative" in the present context includes pharmaceutically acceptable salts, which indicate a salt which is not harmful to the individuals. Such salts include pharmaceutically acceptable basic or acid addition salts as well as pharmaceutically acceptable metal salts, ammonium salts and alkylated ammonium salts. A pharmaceutically acceptable derivative further includes esters and prodrugs, or other precursors of a compound which may be biologically metabolized into the active compound, or crystal forms of a compound.

The term "acid addition salt" is intended to include "pharmaceutically acceptable acid addition salt" which indicates salts which are not harmful to the individual. Acid addition salts include salts of inorganic acids as well as organic acids. Representative examples of suitable inorganic acids include hydrochloric, hydrobromic, hydroiodic, phosphoric, sulfuric, nitric acids and the like. Representative examples of suitable organic acids include formic, acetic, trichloroacetic, trifluoroacetic, propionic, benzoic, cinnamic, citric, fumaric, glycolic, lactic, maleic, malic, malonic, mandelic, oxalic, picric, pyruvic, salicylic, succinic, methanesulfonic, ethanesulfonic, tartaric, ascorbic, pamoic, bismethylene salicylic, ethanedisulfonic, gluconic, citraconic, aspartic, stearic, palmitic, EDTA, glycolic, p-aminobenzoic, glutamic, benzenesulfonic, p-toluenesulfonic acids and the like. Further examples of pharmaceutically acceptable inorganic or organic acid addition salts include the pharmaceutically acceptable salts listed in J. Pharm. Sci. 66, 2, (1977).

The term "therapeutically effective amount" of a compound as used herein refers to an amount sufficient to cure, alleviate, prevent, reduce the risk of, or partially arrest the clinical manifestations of a given disease or disorder and its complications. An amount adequate to accomplish this is defined as "therapeutically effective amount". Effective amounts for each purpose will depend on the severity of the disease or injury as well as the weight and general state of the individual. It will be understood that determining an appropriate dosage may be achieved using routine experimentation, by constructing a matrix of values and testing different points in the matrix, which is all within the ordinary skills of a trained physician or veterinary.

The terms "treatment" and "treating" as used herein refer to the management and care of an individual for the purpose of combating a condition, disease or disorder. The term is intended to include the full spectrum of treatments for a given condition from which the individual is suffering. The individual to be treated is preferably a mammal, in particular a human being. Treatment of animals, such as mice, rats, dogs, cats, horses, cows, sheep and pigs, is, however, also within the scope of the present context. The individuals to be treated can be of various ages.

### Methods of treatment

It is an aspect of the present disclosure to provide an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of treating Gaucher disease (GD) type 1 (GD1) or GD type 3 (GD3).

In some embodiments said active pharmaceutical ingredient is for use in a method of treating Gaucher disease (GD) type 1 (GD1) or GD type 3 (GD3) with a deficient GBA enzyme activity below the lower limit of normal.

In some embodiments said active pharmaceutical ingredient is for use in a method of treating Gaucher disease (GD) type 1 (GD1).

In some embodiments said active pharmaceutical ingredient is for use in a method of treating Gaucher disease (GD) type 1 (GD1) with signs of brain involvement, such as with nonclinical signs of brain involvement.

In some embodiments said active pharmaceutical ingredient is for use in a method of treating Gaucher disease (GD) type 3 (GD3).

In some embodiments said active pharmaceutical ingredient is for use in a method of treating Gaucher disease (GD) type 3 (GD3) with signs of brain involvement, such as with clinical signs of brain involvement.

In some embodiments said active pharmaceutical ingredient is for use in a method of treating Gaucher disease (GD) type 3 (GD3) with at least 1 neurological symptom.

It is an aspect of the present disclosure to provide an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of reducing the clinical composite measure of Gaucher disease (GD) type 1.

It is an aspect of the present disclosure to provide an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of reducing the Clinical GD1 Severity Score.

In some embodiments said active pharmaceutical ingredient is for use in a method of treating Gaucher disease type 1 (GD1) or type 3 (GD3) in a patient of age ≥ 4 and ≤ 60 years.

In some embodiments said active pharmaceutical ingredient is for use in a method of treating Gaucher disease type 1 (GD1) or type 3 (GD3) in a patient of age 4 to 6 years, such as 6 to 8 years, such as 8 to 10 years, such as 10 to 12 years, such as 12 to 14 years, such as 14 to 16 years, such as 16 to 18 years.

In some embodiments said active pharmaceutical ingredient is for use in a method of treating Gaucher disease type 1 (GD1) or type 3 (GD3) in patient with a body weight of ≥ 10 kg.

It is an aspect of the present disclosure to provide an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of treating Gaucher disease type 1 (GD1) or type 3 (GD3), wherein said active pharmaceutical ingredient is administered at 100 mg t.i.d, such as 200 mg t.i.d., such as 400 mg t.i.d.

It is an aspect of the present disclosure to provide an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of treating Gaucher disease type 1 (GD1) or type 3 (GD3), wherein said active pharmaceutical ingredient is administered at 300 mg/day, such as 600 mg/day, such as 1200 mg/day.

In some embodiments said active pharmaceutical ingredient is administered at 100 mg t.i.d, corresponding to 300 mg/day ('Low dosage').

In some embodiments said active pharmaceutical ingredient is administered at 200 mg t.i.d., corresponding to 600 mg/day ('Medium dosage').

In some embodiments said active pharmaceutical ingredient is administered at 400 mg t.i.d, corresponding to 1200 mg/day ('High dosage').

In some embodiments said active pharmaceutical ingredient is administered in weight-adjusted dosages, correlating the Low dosage, Medium dosage and High dosage to the body weight of the patient. The correlation between weight bands and dosages are given herein below.

It is also an aspect of the present disclosure to provide an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of reducing liver size in a patient with Gaucher disease type 1 (GD1) or type 3 (GD3).

It is an aspect of the present disclosure to provide an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of treating hepatomegaly in a patient with Gaucher disease type 1 (GD1) or type 3 (GD3).

It is an aspect of the present disclosure to provide an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of reducing spleen size in a patient with Gaucher disease type 1 (GD1) or type 3 (GD3).

It is an aspect of the present disclosure to provide an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of treating splenomegaly in a patient with Gaucher disease type 1 (GD1) or type 3 (GD3).

It is an aspect of the present disclosure to provide an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of reducing spleen and liver size in a patient with Gaucher disease type 1 (GD1) or type 3 (GD3).

It is an aspect of the present disclosure to provide an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of treating hepatosplenomegaly in a patient with Gaucher disease type 1 (GD1) or type 3 (GD3).

It is an aspect of the present disclosure to provide an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of treating splenomegaly with anaemia and thrombocytopenia, or hepatosplenomegaly with anaemia and thrombocytopenia, in a patient with Gaucher disease type 1 (GD1) or type 3 (GD3).

It is an aspect of the present disclosure to provide an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of treating anaemia in a patient with Gaucher disease type 1 (GD1) or type 3 (GD3).

In one embodiment said patient with Gaucher disease type 1 (GD1) or type 3 (GD3) is anemic.

It is an aspect of the present disclosure to provide an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of increasing haemoglobin in a patient with Gaucher disease type 1 (GD1) or type 3 (GD3).

In one embodiment said method of increasing haemoglobin is a method of increasing haemoglobin in a patient with Gaucher disease type 1 (GD1) or type 3 (GD3) who is anemic.

In one embodiment said method of increasing haemoglobin is a method of increasing haemoglobin in an anemic patient with Gaucher disease type 1 (GD1) or type 3 (GD3).

In one embodiment said treatment increases haemoglobin at a dosage of 600 mg/day (200 mg t.i.d.) or above; such as at a dosage of 600 - 900 mg/day (200-300 mg t.i.d.) or 900-1200 mg/day (300-400 mg t.i.d.); such as increases haemoglobin at a dosage of 600 mg/day (200 mg t.i.d.) or 1200 mg/day (400 mg t.i.d.).

In one embodiment said treatment increases haemoglobin at a dosage of 600 mg/day (200 mg t.i.d.) or above; such as at a dosage of 600 - 900 mg/day (200-300 mg t.i.d.) or 900-1200 mg/day (300-400 mg t.i.d.); such as increases haemoglobin at a dosage of 600 mg/day (200 mg t.i.d.) or 1200 mg/day (400 mg t.i.d.), wherein said dosages are weight-adjusted (cf. herein elsewhere).

It is an aspect of the present disclosure to provide an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of reducing serum chitotriosidase activity in a patient with Gaucher disease type 1 (GD1) or type 3 (GD3).

It is an aspect of the present disclosure to provide an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of increasing glycosylsphingosine (lyso-Gb1) in a patient with Gaucher disease type 1 (GD1) or type 3 (GD3).

In one embodiment said Gaucher disease (GD) is GD Type 1 (GD1) , or GD type 3 (GD3).

In one embodiment said Gaucher disease (GD) is GD Type 1 (GD1).

In one embodiment said Gaucher disease (GD) is GD type 3 (GD3).

In one embodiment said treatment dose-dependently reduces liver size.

In one embodiment said treatment dose-dependently reduces spleen size.

In one embodiment said treatment dose-dependently reduces chitotriosidase activity.

In one embodiment said treatment time-dependently increases haemoglobin.

It is an aspect of the present disclosure to provide an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of treating Gaucher disease (GD) type 1 (GD1) or type 3 (GD3), wherein the maximum predicted AUC_{0-8 hrs} (High dose) is within about 80.00% to about 125.00% of a AUC_{0-8 hrs} of 6 to 9 µg·h/mL, after administration of a single dose.

In some embodiments, the maximum predicted AUC_{0-8 hrs} (High dose) is within about 80.00% to about 125.00% of a AUC_{0-8 hrs} of 6 to 9 µg·h/mL, such as about 6 to 6.5, such as about 6.5 to 7, such as about 7 to 7.5, such as about 7.5 to 8, such as about 8 to 8.5 , such as about 8.5 to 9 µg·h/mL, after administration of a single dose.

In some embodiments, the maximum predicted AUC_{0-8 hrs} (High dose) is within about 80.00% to about 125.00% of a AUC_{0-8 hrs} of 6 to 9 µg·h/mL, such as about 6 to 6.5, such as about 6.5 to 7, such as about 7 to 7.5, such as about 7.5 to 8, such as about 8 to 8.5 , such as about 8.5 to 9 µg·h/mL, after administration of a single dose,
wherein said single dose is 100 mg for a patient with a body weight of 10 kg to ≤ 30 kg, and/or
wherein said single dose is 200 mg for a patient with a body weight of 30 kg to ≤ 50 kg, and/or
wherein said single dose is 300 mg for a patient with a body weight of 50 kg to ≤ 70 kg, and/or
wherein said single dose is 400 mg for a patient with a body weight of ≥ 70 kg.

In some embodiments there is provided an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of treating Gaucher disease (GD) type 1 (GD1) or type 3 (GD3), wherein AUC₀₋₈ hrs, steady state is within about 80.00% to about 125.00% of a AUC₀₋₈ hrs, steady state of about 1000 h·ng/mL, after administration of 300 mg/day.

In some embodiments the AUC_{0-8 hrs, steady state} is within about 80.00% to about 125.00% of a AUC_{0-8 hrs, steady state} of about 700 to 1300 h·ng/mL, such as of about 800 to 1200 h·ng/mL, such as of about 900 to 1100 h·ng/mL, after administration of 300 mg/day.

In some embodiments there is provided an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of treating Gaucher disease (GD) type 1 (GD1) or type 3 (GD3), wherein AUC₀₋₈ hrs, steady state is within about 80.00% to about 125.00% of a AUC₀₋₈ hrs, steady state of about 2000 h·ng/mL, after administration of 600 mg/day.

In some embodiments the AUC_{0-8 hrs, steady state} is within about 80.00% to about 125.00% of a AUC_{0-8 hrs, steady state} of about 1700 to 2300 h·ng/mL, such as about 1800 to 2200 h·ng/mL, such as about 1900 to 2100 h·ng/mL after administration of 600 mg/day.

In some embodiments there is provided an active pharmaceutical ingredient selected from *N*-[2-hydroxy-3-(1-piperidinyl)-propoxy]-pyridine-1-oxide-3-carboximidoyl chloride, its stereoisomers and the acid addition salts thereof, for use in a method of treating Gaucher disease (GD) type 1 (GD1) or type 3 (GD3), wherein AUC_{0-8 hrs, steady state} is within about 80.00% to about 125.00% of a AUC_{0-8 hrs, steady state} of about 5100 h·ng/mL, after administration of 1200 mg/day.

In some embodiments the AUC_{0-8 hrs, steady state} is within about 80.00% to about 125.00% of a AUC_{0-8 hrs, steady state} of about 4500 to 5700 h·ng/mL, such as 4500 to 4600 h·ng/mL, such as 4600 to 4700 h·ng/mL, such as 4700 to 4800 h·ng/mL, such as 4800 to 4900 h·ng/mL, such as 4900 to 5000 h·ng/mL, such as 5000 to 5100 h·ng/mL, such as 5100 to 5200 h·ng/mL, such as 5200 to 5300 h·ng/mL, such as 5300 to 5400 h·ng/mL, such as 5400 to 5500 h·ng/mL, such as 5500 to 5600 h·ng/mL, such as 5600 to 5700 h·ng/mL after administration of 1200 mg/day.

In one embodiment said treatment is prophylactic, curative or ameliorating. In one particular embodiment, said treatment is prophylactic. In another embodiment, said treatment is curative. In a further embodiment, said treatment is ameliorating.

In some embodiments, the individual has previously been treated for Gaucher disease, such as with enzyme replacement therapy, substrate replacement therapy, blood transfusion and/or splenectomy. In some embodiments, the individual has previously been treated with enzyme replacement therapy and/or substrate replacement therapy. In some embodiments, the individual has not been treated for Gaucher disease, such as with enzyme replacement therapy, substrate replacement therapy, blood transfusion and/or splenectomy, within the preceding 4 months, such as within the preceding 3 months, such as within the preceding 2 months, such as within the preceding month, before the start of treatment. In some embodiments, the individual has not been treated with enzyme replacement therapy and/or substrate replacement therapy within the preceding 4 months, such as within the preceding 3 months, such as within the preceding 2 months, such as within the preceding month, before the start of treatment. In some embodiments, the individual is naïve to treatment for Gaucher disease, such as with enzyme replacement therapy, substrate replacement therapy, blood transfusion and/or splenectomy. In some embodiments, the individual is naïve to treatment with enzyme replacement therapy and/or substrate replacement therapy

The individual to be treated is preferably a mammal, in particular a human being. Treatment of animals, such as mice, rats, dogs, cats, horses, cows, sheep and pigs, is, however, also within the scope of the present disclosure. The individual to be treated can be of various ages, including infant, child, adolescent and adult. In a preferred embodiment, the individual as used herein is a human being, male or female, of any age.

An "individual in need thereof" refers to a diseased individual who may benefit from the present disclosure, wherein said disease is Gaucher disease type 1 (GD1) or type 3 (GD3). A patient is considered an individual in need thereof; and a patient with Gaucher disease type 1 (GD1) or type 3 (GD3) is considered as a patient in need thereof. The terms "individual" and "patient" may be used interchangeably herein.

### Arimoclomol

The present disclosure provides an active pharmaceutical ingredient selected from *N-*[2-hydroxy-3-(1-piperidinyl)-propoxy]-pyridine-1-oxide-3-carboximidoyl chloride, its stereoisomers and the acid addition salts thereof, for the present purposes.

In some embodiments, the active pharmaceutical ingredient is the racemate of *N*-[2-hydroxy-3-(1-piperidinyl)-propoxy]-pyridine-1-oxide-3-carboximidoyl chloride.

In some embodiments, the active pharmaceutical ingredient is an optically active stereoisomer of *N*-[2-hydroxy-3-(1-piperidinyl)-propoxy]-pyridine-1-oxide-3-carboximidoyl chloride.

In some embodiments, the active pharmaceutical ingredient is an enantiomer of *N*-[2-hydroxy-3-(1-piperidinyl)-propoxy]-pyridine-1-oxide-3-carboximidoyl chloride.

In some embodiments, the active pharmaceutical ingredient is selected from the group consisting of (+)-(*R*)-*N*-[2-hydroxy-3-(1-piperidinyl)-propoxy]-pyridine-1-oxide-3-carboximidoyl chloride and (-)-(*S*)-*N*-[2-hydroxy-3-(1-piperidinyl)-propoxy]-pyridine-1-oxide-3-carboximidoyl chloride.

In some embodiments, the active pharmaceutical ingredient is selected from the group consisting of (*Z*)-(*R*)-*N*-[2-hydroxy-3-(1-piperidinyl)-propoxy]-pyridine-1-oxide-3-carboximidoyl chloride, (*E*)-(*R*)-*N*-[2-hydroxy-3-(1-piperidinyl)-propoxy]-pyridine-1-oxide-3-carboximidoyl chloride, (*Z*)-(*S*)-*N*-[2-hydroxy-3-(1-piperidinyl)-propoxy]-pyridine-1-oxide-3-carboximidoyl chloride, and (*E*)-(*S*)-*N*-[2-hydroxy-3-(1-piperidinyl)-propoxy]-pyridine-1-oxide-3-carboximidoyl chloride.

In some embodiments, the active pharmaceutical ingredient is an acid addition salt of *N*-[2-hydroxy-3-(1-piperidinyl)-propoxy]-pyridine-1-oxide-3-carboximidoyl chloride.

In some embodiments, the active pharmaceutical ingredient is selected from the group consisting of *N*-[2-hydroxy-3-(1-piperidinyl)-propoxy]-pyridine-1-oxide-3-carboximidoyl chloride citrate, and *N*-[2-hydroxy-3-(1-piperidinyl)-propoxy]-pyridine-1-oxide-3-carboximidoyl chloride maleate.

In some embodiments, the active pharmaceutical ingredient is selected from the group consisting of (+)-(*R*)-*N*-[2-hydroxy-3-(1-piperidinyl)-propoxy]-pyridine-1-oxide-3-carboximidoyl chloride citrate, (-)-(*S*)-*N*-[2-hydroxy-3-(1-piperidinyl)-propoxy]-pyridine-1-oxide-3-carboximidoyl chloride citrate, (+)-(*R*)-*N*-[2-hydroxy-3-(1-piperidinyl)-propoxy]-pyridine-1-oxide-3-carboximidoyl chloride maleate and (-)-(*S*)-*N*-[2-hydroxy-3-(1-piperidinyl)-propoxy]-pyridine-1-oxide-3-carboximidoyl chloride maleate.

In some embodiments, the active pharmaceutical ingredient is selected from the group consisting of (*Z*)-(*R*)-*N*-[2-hydroxy-3-(1-piperidinyl)-propoxy]-pyridine-1-oxide-3-carboximidoyl chloride citrate, (*E*)-(*R*)-*N*-[2-hydroxy-3-(1-piperidinyl)-propoxy]-pyridine-1-oxide-3-carboximidoyl chloride citrate, (*Z*)-(*S*)-*N*-[2-hydroxy-3-(1-piperidinyl)-propoxy]-pyridine-1-oxide-3-carboximidoyl chloride citrate, (*E*)-(*S*)-*N*-[2-hydroxy-3-(1-piperidinyl)-propoxy]-pyridine-1-oxide-3-carboximidoyl chloride citrate, (*Z*)-(*R*)-*N*-[2-hydroxy-3-(1-piperidinyl)-propoxy]-pyridine-1-oxide-3-carboximidoyl chloride maleate, (*E*)-(*R*)-*N*-[2-hydroxy-3-(1-piperidinyl)-propoxy]-pyridine-1-oxide-3-carboximidoyl chloride maleate, (*Z*)-(*S*)-*N*-[2-hydroxy-3-(1-piperidinyl)-propoxy]-pyridine-1-oxide-3-carboximidoyl chloride maleate and (*E*)-(*S*)-*N*-[2-hydroxy-3-(1-piperidinyl)-propoxy]-pyridine-1-oxide-3-carboximidoyl chloride maleate.

In some embodiments, the active pharmaceutical ingredient is arimoclomol.

In some embodiments, the active pharmaceutical ingredient is arimoclomol (free base), (+)-(*R*)-*N*-[2-hydroxy-3-(1-piperidinyl)-propoxy]-pyridine-1-oxide-3-carboximidoyl chloride. Arimoclomol and its preparation is disclosed e.g. in WO 97/16439, WO 00/050403 and WO 01/79174. In some embodiments, arimoclomol is the citrate salt formulation of the free base, i.e. (+)-(*R*)-*N*-[2-hydroxy-3-(1-piperidinyl)-propoxy]-pyridine-1-oxide-3-carboximidoyl chloride citrate (also known as BRX-345). Arimoclomol free base has the structure as shown in formula I:

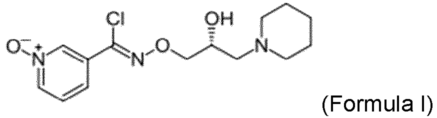

### Administration and dosage

The active pharmaceutical ingredient, or a composition comprising the same as defined herein, is in some embodiments administered to an individual in need thereof in pharmaceutically effective doses or in a therapeutically effective amount.

In some embodiment, the active pharmaceutical ingredient is administered one or several times per day, such as from 1 to 3 times per day, such as from 1 to 2 times per day, such as from 2 to 3 times per day.

In some embodiments, the active pharmaceutical ingredient is administered daily, such as once daily, such as twice daily, such as three times daily (t.i.d).

In a preferred embodiment, the active pharmaceutical ingredient is administered three times daily (t.i.d).

In some embodiments, the active pharmaceutical ingredient is administered for 1 week such as more than 1 week, such as 2 weeks or more than 2 weeks, such as 3 weeks or more than 3 weeks, such as 4 weeks or more than 4 weeks, such as for 1 month or more than 1 month, such as 2 months or more than 2 months, such as 3 months or more than 3 months, such as 4 months or more than 4 months, such as 5 months or more than 5 months, such as 6 months or more than 6 months, such as 7 months or more than 7 months, such as 8 months or more than 8 months, such as 9 months or more than 9 months, such as 9 months or more than 9 months, such as 10 months or more than 10 months, such as 11 months or more than 11 months, or such as for 1 year or more than 1 year.

In some embodiments, the active pharmaceutical ingredient is administered for at least 6 months. In some embodiments, the active pharmaceutical ingredient is administered for at least 9 months, such as for at least 1 year, such as for at least 18 months, such as for at least 2 years, such as for at least 30 months, such as for at least 3 years, such as for at least 4 years, such as for at least 5 years, such as for at least 6 years, such as for at least 7 years, such as for at least 8 years, such as for at least 9 years, such as for at least 10 years or more.

In some embodiments, the dosages are calculated on the basis of the arimoclomol free base ((+)-(*R*)-*N*-[2-hydroxy-3-(1-piperidinyl)-propoxy]-pyridine-1-oxide-3-carboximidoyl chloride).

In some embodiments, the dosages are calculated on the basis of the arimoclomol citrate salt ((+)-(*R*)-*N*-[2-hydroxy-3-(1-piperidinyl)-propoxy]-pyridine-1-oxide-3-carboximidoyl chloride citrate).

If nothing else is specified, the dosage of the active pharmaceutical ingredient as disclosed herein is indicated as the citrate salt, and the conversion scheme between the citrate salt and the free base is as follows:

**Conversion factor from citrate to base: [citrate dose] * 0.620241**

| ***Dose Arimoclomol citrate [mg]*** | ***Dose Arimoclomol free base [mg]*** |
|---|---|
| 25 | 16 |
| 50 | 31 |
| 75 | 47 |
| 100 | 62 |
| 150 | 93 |
| 200 | 124 |
| 300 | 186 |
| 400 | 248 |
| 600 | 372 |
| 800 | 496 |
| 1200 | 744 |
| 1800 | 1116 |

In some embodiments, the active pharmaceutical ingredient is arimoclomol citrate salt ((+)-(*R*)-*N*-[2-hydroxy-3-(1-piperidinyl)-propoxy]-pyridine-1-oxide-3-carboximidoyl chloride citrate).

In some embodiments, the active pharmaceutical ingredient is administered at a per administration dose of about 25 mg to about 800 mg, such as about 100 mg to about 800 mg. In some embodiments, the active pharmaceutical ingredient is administered at a per administration dose of about 25 mg to about 50 mg, such as about 50 mg to about 75 mg, such as about 75 mg to about 100 mg, such as about 100 mg to about 150 mg, such as about 150 mg to about 200 mg, such as about 200 mg to about 300 mg, such as about 300 mg to about 400 mg, such as about 400 mg to about 500 mg, such as about 500 mg to about 600 mg, such as about 600 mg to about 700 mg, such as about 700 mg to about 800 mg, preferably wherein the active pharmaceutical ingredient is administered three times daily.

In some embodiments, the active pharmaceutical ingredient is administered at a per administration dose of about 25 mg, such as about 50 mg, such as about 75 mg, such as about 100 mg, such as about 150 mg, such as about 200 mg, for example 300 mg, such as 400 mg, for example 500 mg, such as 600 mg, for example 700 mg, for example 800 mg, preferably wherein the active pharmaceutical ingredient is administered three times daily.

In some embodiments, the active pharmaceutical ingredient is administered at a per administration dose of about 100 mg, such as about 200 mg, for example about 400 mg, preferably wherein first active pharmaceutical ingredient is administered three times daily. In some embodiments said per administration dose is weight-adjusted.

In some embodiments, the active pharmaceutical ingredient is administered at 100 mg t.i.d., such as 200 mg t.i.d., for example 400 mg t.i.d. In some embodiments said dose is weight-adjusted.

In some embodiments, the active pharmaceutical ingredient is administered at a daily dosage of about 100 mg/day to about 200 mg/day, such as about 200 mg/day to about 300 mg/day, such as about 300 mg/day to about 400 mg/day, such as about 400 mg/day to about 500 mg/day, such as about 500 mg/day to about 600 mg/day, such as about 700 mg/day to about 800 mg/day, such as about 800 mg/day to about 900 mg/day, such as about 900 mg/day to about 1000 mg/day, such as about 1000 mg/day to about 1100 mg/day, such as about 1100 mg/day to about 1200 mg/day.

In preferred embodiments, the active pharmaceutical ingredient is administered at a daily dosage of 300 mg/day, such as 600 mg/day, for example 1200 mg/day.

In preferred embodiments, the active pharmaceutical ingredient is administered orally. In some embodiments, the active pharmaceutical ingredient is administered via a gastric tube.

In some embodiments, the active pharmaceutical ingredient is administered in hard capsules of 25 mg, 50 mg, or 100 mg.

In some embodiments, the active pharmaceutical ingredient is administered to a patient with Gaucher disease type 1 (GD1) or type 3 (GD3) having a body weight of ≥ 10 kg.

In some embodiments, the active pharmaceutical ingredient is administered to a patient with Gaucher disease type 1 (GD1) or type 3 (GD3) in doses based on patient body weight.

In some embodiments, the active pharmaceutical ingredient is administered to a patient with Gaucher disease type 1 (GD1) or type 3 (GD3) in weight-adjusted dosages.

In some embodiments, the active pharmaceutical ingredient is administered at 100 mg t.i.d., corresponding to a dosage of 300 mg/day. This is also referred to a 'Low' dosage.

In some embodiments, the active pharmaceutical ingredient is administered at 200 mg t.i.d., corresponding to a dosage of 400 mg/day. This is also referred to a 'Medium' dosage.

In some embodiments, the active pharmaceutical ingredient is administered at 400 mg t.i.d., corresponding to a dosage of 1200 mg/day. This is also referred to a 'High' dosage.

In some embodiments, the active pharmaceutical ingredient is administered to a patient with Gaucher disease type 1 (GD1) or type 3 (GD3) having a body weight of 10 kg to ≤ 30 kg.

In some embodiments, the active pharmaceutical ingredient is administered at 25 mg t.i.d., corresponding to a dosage of 75 mg/day to a patient with Gaucher disease type 1 (GD1) or type 3 (GD3) having a body weight of 10 kg to ≤ 30 kg ('Low dosage').

In some embodiments, the active pharmaceutical ingredient is administered at 50 mg t.i.d., corresponding to a dosage of 150 mg/day to a patient with Gaucher disease type 1 (GD1) or type 3 (GD3) having a body weight of 10 kg to ≤ 30 kg ('Medium dosage').

In some embodiments, the active pharmaceutical ingredient is administered at 100 mg t.i.d., corresponding to a dosage of 300 mg/day to a patient with Gaucher disease type 1 (GD1) or type 3 (GD3) having a body weight of 10 kg to ≤ 30 kg ('High dosage').

In some embodiments, the active pharmaceutical ingredient is administered to a patient with Gaucher disease type 1 (GD1) or type 3 (GD3) having a body weight of 30 kg to ≤ 50 kg.

In some embodiments, the active pharmaceutical ingredient is administered at 50 mg t.i.d., corresponding to a dosage of 150 mg/day to a patient with Gaucher disease type 1 (GD1) or type 3 (GD3) having a body weight of 30 kg to ≤ 50 kg ('Low dosage').

In some embodiments, the active pharmaceutical ingredient is administered at 100 mg t.i.d., corresponding to a dosage of 300 mg/day to a patient with Gaucher disease type 1 (GD1) or type 3 (GD3) having a body weight of 30 kg to ≤ 50 kg ('Medium dosage').

In some embodiments, the active pharmaceutical ingredient is administered at 200 mg t.i.d., corresponding to a dosage of 600 mg/day to a patient with Gaucher disease type 1 (GD1) or type 3 (GD3) having a body weight of 30 kg to ≤ 50 kg ('High dosage').

In some embodiments, the active pharmaceutical ingredient is administered to a patient with Gaucher disease type 1 (GD1) or type 3 (GD3) having a body weight of 50 kg to ≤ 70 kg.

In some embodiments, the active pharmaceutical ingredient is administered at 75 mg t.i.d., corresponding to a dosage of 225 mg/day to a patient with Gaucher disease type 1 (GD1) or type 3 (GD3) having a body weight of 50 kg to ≤ 70 kg ('Low dosage').

In some embodiments, the active pharmaceutical ingredient is administered at 150 mg t.i.d., corresponding to a dosage of 450 mg/day to a patient with Gaucher disease type 1 (GD1) or type 3 (GD3) having a body weight of 50 kg to ≤ 70 kg ('Medium dosage').

In some embodiments, the active pharmaceutical ingredient is administered at 300 mg t.i.d., corresponding to a dosage of 900 mg/day to a patient with Gaucher disease type 1 (GD1) or type 3 (GD3) having a body weight of 50 kg to ≤ 70 kg ('High dosage').

In some embodiments, the active pharmaceutical ingredient is administered to a patient with Gaucher disease type 1 (GD1) or type 3 (GD3) having a body weight of ≥ 70 kg.

In some embodiments, the active pharmaceutical ingredient is administered at 100 mg t.i.d., corresponding to a dosage of 300 mg/day to a patient with Gaucher disease type 1 (GD1) or type 3 (GD3) having a body weight of ≥ 70 kg ('Low dosage').

In some embodiments, the active pharmaceutical ingredient is administered at 200 mg t.i.d., corresponding to a dosage of 600 mg/day to a patient with Gaucher disease type 1 (GD1) or type 3 (GD3) having a body weight of ≥ 70 kg ('Medium dosage').

In some embodiments, the active pharmaceutical ingredient is administered at 400 mg t.i.d., corresponding to a dosage of 1200 mg/day to a patient with Gaucher disease type 1 (GD1) or type 3 (GD3) having a body weight of ≥ 70 kg ('High dosage').

In some embodiments, the active pharmaceutical ingredient is administered to a patient with Gaucher disease type 1 (GD1) or type 3 (GD3) of age of age ≥ 4 and ≤ 60 years.

In some embodiments, the active pharmaceutical ingredient is administered to a patient with Gaucher disease type 1 (GD1) or type 3 (GD3) in doses based on age.

In some embodiments, the active pharmaceutical ingredient is administered at 100 mg t.i.d., corresponding to a dosage of 300 mg/day to a patient with Gaucher disease type 1 (GD1) or type 3 (GD3) aged > 18 years.

In some embodiments, the active pharmaceutical ingredient is administered at 200 mg t.i.d., corresponding to a dosage of 600 mg/day to a patient with Gaucher disease type 1 (GD1) or type 3 (GD3) aged > 18 years.

In some embodiments, the active pharmaceutical ingredient is administered at 400 mg t.i.d., corresponding to a dosage of 1200 mg/day to a patient with Gaucher disease type 1 (GD1) or type 3 (GD3) aged > 18 years.

In some embodiments, the active pharmaceutical ingredient is arimoclomol citrate salt ((+)-(*R*)-*N*-[2-hydroxy-3-(1-piperidinyl)-propoxy]-pyridine-1-oxide-3-carboximidoyl chloride citrate). In some embodiments, the dosages of the active pharmaceutical ingredient is calculated based on the arimoclomol citrate salt ((+)-(*R*)-*N*-[2-hydroxy-3-(1-piperidinyl)-propoxy]-pyridine-1-oxide-3-carboximidoyl chloride citrate).

### Formulation

Whilst it is possible for the active pharmaceutical ingredient to be administered as the raw chemical, it is in some embodiments preferred to present them in the form of a pharmaceutical formulation. Accordingly, also provided herewith is a composition, such as a pharmaceutical composition, i.e. a pharmaceutically safe composition, comprising the active pharmaceutical ingredients as defined herein. The composition in some embodiments further comprises one or more pharmaceutically and/or physiologically acceptable diluents, carriers and/or excipients.

In some embodiments, the composition is formulated for oral administration, such as in the form of tablets or capsules, or such as an oral powder, such as an oral powder suitable for suspension in a liquid, or such as as a suspension for oral administration.

Pharmaceutical compositions containing the active pharmaceutical ingredients of the present disclosure may be prepared by conventional techniques.

### Examples

### Example 1 - Multicentre, double-blinded, randomised, placebo-controlled trial of arimoclomol in patients diagnosed with Gaucher Disease Type 1 or 3

### Materials and methods

### Methodology/Trial Design

This is a placebo-controlled, randomised, double-blinded trial evaluating the response of 3 dose levels of arimoclomol on various pharmacodynamic (PD) biomarkers in blood and cerebrospinal fluid (CSF) as indicators of enhanced glucocerebrosidase (GBA) enzyme activity in GD1 and GD3.

The purpose of the trial was to assess if arimoclomol can increase the GBA enzyme activity in GD patients in a dose-related manner. The change in serum values of well-established biomarkers linked to GBA enzyme activity, as well as change in haemoglobin and platelet counts was assessed during a double-blinded 6-month period and compared to placebo. As both GD1 and GD3 patients have signs of brain involvement (albeit only nonclinical for GD1 patients), the same panel of biomarkers was evaluated in the CSF of GD1 and GD3 patients.

Eligible GD1 and GD3 patients were randomised into 4 groups on a 1:1:1:1 basis to receive arimoclomol 300 mg/day, arimoclomol 600 mg/day, arimoclomol 1200 mg/day, or placebo treatment (3 times daily [t.i.d] dosing).

Paediatric patients were administered a corresponding dose based on body weight in kilograms. Adult patients, randomised into the trial before the effective date of this protocol received a fixed dose and adult patients randomised into the trial after the effective date of this protocol were administered an IMP dose based on their body weight. Patients were stratified according to GDtype (Type 1 or Type 3). Patients who were randomised to receive placebo were further randomised to receive arimoclomol 300 mg/day, 600mg/day, or 1200 mg/day (provided there are no safety concerns) on a 1:1:1 basis during the open-label extension phase of the trial.

In the event of intolerable adverse events (AEs) according to the Investigator's judgement, the patient was dispensed a reduced dose of investigational medicinal product (IMP) until Visit 3. The patient stayed at the reduced dose for the remainder of the study.

All patients underwent an arimoclomol single-dose (100-mg single dose for the 300-mg/day treatment group, 200-mg single dose for the arimoclomol 600-mg/day treatment group, 400-mg single dose for the arimoclomol 1200-mg/day treatment group, or corresponding dose per kg for paediatric patients prior to the effective date of this protocol. Single dose pharmacokinetics (PK) was done with corresponding dose per kg for all patients randomised after the effective date of this protocol) pharmacokinetic (PK) evaluation at Visit 2 following randomization to confirm the suitability (in terms of target investigational medicinal product (AUC0-8 - area under the curve [AUC] from time 0 to 8 hours [AUC0-8]) of the selected doses in the trial. Following the single dose, blood samples for PK analysis were collected at predose, and 0.5, 1, 2, 4, and 8 hours postdose. Patients randomised to the placebo group were administered the dose intended for the extension phase. The corresponding data was evaluated by an independent assessor to verify the AUC0-8. Patients then commenced continuous dosing of IMP immediately as per the trial randomisation. The IMP was administered three times daily (t.i.d).

In the event that the AUC was above the target, or an unexpected profile which could potentially impact the safety of the patient was obtained, and the patient required a dose reduction, the patient was dispensed a reduced dose of IMP (blinded) at Visit 3. The dose level was recommended by the independent assessor. Additional blood samples for quantitative population PK analysis of arimoclomol were collected predose and at 0.5 hours postdose at Visit 5, and predose and 1.5 hours postdose at Visit 7.

Cerebrospinal fluid samples for quantitative population PK analyses of arimoclomol were collected predose at Visit 5, and postdose following the first daily dose at Visit 7. The population PK analysis and PK/PD modelling results were reported separately. Following the proof-of-concept phase, all patients were offered the opportunity to continue into an open-label extension phase, during which they attended the site 1 month after the end of blinded phase visit and every 6 months after the end of blinded phase visit. During this open-label extension phase, patients who had received arimoclomol during the proof-of-concept phase of the trial continued on the same arimoclomol dose they had been assigned to, while patients who had received placebo received arimoclomol 300 mg/day, 600 mg/day, or 1200 mg/day (adult patients) or corresponding weight-adjusted dose (paediatric patients). All patients randomised after the effective date of this protocol were administered an IMP dose based on body weight.

### Trial Population and Number of Patients

Male and female patients between 4 and 60 years with a diagnosis of GD, either Type 1 or Type 3 constituted the trial population. It was anticipated that 40 patients would be enrolled/randomised into the trial.

All randomised patients population: All patients who completed screening procedures and were randomly assigned to a treatment group.

Intent-to-treat (ITT) population: All randomised patients who received at least 1 dose of IMP and had valid serum chitotriosidase activity (primary endpoint) at baseline and any post-baseline visits.

Per-protocol (PP) population: All randomised patients who received at least 80% of IMP in the treatment phase, had at least 1 chitotriosidase (CLS) measurement at baseline (Visit 1 or 2) and at 6 months (Visit 5), had plasma or serum chitotriosidase activity greater than 3 times the upper limit of normal applicable for the local laboratory test procedure (historical data were acceptable), and were confirmed as GD Type 1 or 3 in the genetic analysis at Visit 4.

Blood/CSF population: All randomised patients who had a blood and CSF measurement at Visits 5 and 7 to correlate arimoclomol concentration in blood and CSF with chitotriosidase activity.

All Patients Treated Set: All patients who received at least 1 dose of IMP.

Efficacy was analysed using the ITT population and PP population, and safety was analysed using the All Patients Treated Set (unless otherwise specified).

### Diagnosis and Main Criteria for Inclusion:

The following were the main inclusion criteria:
1. The patient or the legally authorised representative is able to read and understand the informed consent form.
2. The patient or the legally authorised representative has signed the informed consent form.
3. A diagnosis of GD, either Type 1 or Type 3, with a deficient GBA enzyme activity below the lower limit of normal. Note that the local laboratory test result was used at inclusion.
4. For GD3, at least 1 neurological symptom.
5. Age ≥ 4 and ≤ 60 years at the time of enrolment.
6. Plasma or serum chitotriosidase activity greater than 3 times the upper limit of normal applicable for the local laboratory test procedure. Historical data are acceptable.
7. Either naïve to treatment for GD or has not received treatment (investigational or authorised/approved such as enzyme replacement therapy or substrate reduction therapy, also including procedures such as blood transfusions and splenectomy) for GD within 4 months prior to trial entry.
8. Ability to comply with the protocol-specified procedures/evaluations and scheduled visits (if platelet counts are< 50000 platelets per microlitre of circulating blood lumbar puncture was not performed).
9. Ability to travel to the investigational clinical trial site repeatedly (screening, baseline, 1, 3, 6, 7, 9 and 12 months, then every 6 months during the remaining time of the extension part) for evaluation and follow-up.
10. All sexually active female patients of child-bearing potential (postmenarcheal) must use highly effective contraception during the trial and until 1 week after the last dose of IMP. Highly effective birth control methods include: Combined (oestrogen and progestogen containing) hormonal contraception associated with inhibition of ovulation (oral, intravaginal, or transdermal);progestogen-only hormonal contraception associated with inhibition of ovulation (oral, injectable, or implantable); intrauterine device; intrauterine hormone-releasing system; bilateral tubal occlusion; and vasectomised partner. All sexually active male patients with female partners of child-bearing potential (postmenarcheal) must use a condom with or without spermicide in addition to the birth control used by their partners during the trial and until 3 months after the last dose of IMP.

### Exclusion criteria

The following were the main exclusion criteria:
1. Recipient of a liver transplant or planned liver transplantation during the course of the trial.
2. Splenectomy within 4 months of trial entry or planned splenectomy during the course of the trial.
3. Aspartate aminotransferase and/or alanine aminotransferase greater than 3 times the upper limit of normal for age and sex [central laboratory assessment]).
4. Serum creatinine level greater than 1.5 times the upper limit of normal (central laboratory assessment).
5. The patient has received any IMP within 30 days prior to trial entry (Note that IMP for GD should not have been taken within the last 4 months prior to enrolment, as per entry criterion #7).
6. The patient is pregnant and/or breastfeeding.
7. If, in the opinion of the Principal Investigator, the patient has a clinical condition that is not compatible with the requirements of this protocol, such as confirmed history of serious adverse reaction to sedation or anaesthesia (if sedation is necessary for lumbar puncture), uncontrolled severe epileptic seizures, and/or severe malnutrition.
8. Body weight < 10 kg

### Test Product, Dose, and Dosage Form

Arimoclomol was administered in hard capsules of 25 mg, 50 mg, and 100 mg.

### Reference Therapy, Dose, and Dosage Form

Placebo: The placebo capsule was visually indistinguishable from the arimoclomol capsule in size and appearance. The placebo was presented with an identical weight capsule fill in identical white hard capsules and packaged and labelled as described for arimoclomol. The excipient composition, texture, appearance, solubility, smell, and flavour of the placebo were carefully matched to mask the identity of the active capsule (arimoclomol).

The IMP (arimoclomol and placebo) was shipped to the centres at 2°C to 25°C and stored at ≤30°C.

### Mode of Administration

The IMP (arimoclomol or placebo) was administered orally t.i.d. If required, the IMP could be dispersed in 20 mL (i.e., 2 tablespoons) of liquid (apple juice, water, or milk) or in 1 tablespoon of soft food (yoghurt or apple sauce). In the dispersed state, the IMP could also be administered via a gastric tube (as applicable). For full administration, the tube was flushed with 5 mL water.

### Dose and regimen

For patients randomised prior to the effective date of this protocol doses in mg were given t.i.d. based on patient weight (see Table 1 below). For paediatric patients, doses were administered according to body weight as shown in Table 1, below. Adult patients (≥ 18 years) were provided with fixed doses as shown in Table 1, below.

**Table 1 - Dose and regimen for patients during the trial (patients randomised prior to the effective date of this protocol).**

| **Patient Weight** | **"Low" 300 mg/d t.i.d Dosing** | **"Medium" 600 mg/d t.i.d Dosing** | **"High" 1200 mg/d t.i.d Dosing** | **Maximum predicted AUC₀₋₈ (µg•h/mL) (high-dose)** | **Placebo t.i.d** |
|---|---|---|---|---|---|
| 10 to 29.9 kg | 25 mg (1 × 25 mg capsule) | 50 mg (1 × 50 mg capsule) | 100 mg (1 × 100 mg capsule) | 7.8 | 1 capsule |
| 30 to 49.9 kg | 50 mg (2 × 25 mg capsules) | 100 mg (2 × 50 mg capsules) | 200 mg (2 × 100 mg capsules) | 6.9 | 2 capsules |
| 50 to 69.9 kg | 75 mg (3 × 25 mg capsules) | 150 mg (3 × 50 mg capsules) | 300 mg (3 × 100 mg capsules) | 7.0 | 3 capsules |
| > 70 kg or adult (> 18 years) | 100 mg (4 × 25 mg capsules) | 200 mg (4 × 50 mg capsules) | 400 mg (4 × 100 mg capsules) | 7.3 | 4 capsules |

| | | | | | |
|---|---|---|---|---|---|
| Abbreviations: AUC₀₋₈ = area under the curve from time 0 to 8 hours; d = day: t.i.d = 3-times daily. | | | | | |

All patients including adult patients randomised into the trial after the effective date of this protocol were administered an IMP dose based on their body weight, as shown in table 2, below.

**Table 2 - Dose and regimen for patients during the trial (patients randomised after the effective date of this protocol).**

| **Patient Weight** | **"Low" 300 mg/d t.i.d Dosing** | **"Medium" 600 mg/d t.i.d Dosing** | **"High" 1200 mg/d t.i.d Dosing** | **Maximum predicted AUC₀₋₈ (µg•h/mL) (high-dose)** | **Placebo t.i.d** |
|---|---|---|---|---|---|
| 10 to 29.9 kg | 25 mg (1 × 25 mg capsule) | 50 mg (1 × 50 mg capsule) | 100 mg (1 × 100 mg capsule) | 7.8 | 1 capsule |
| 30 to 49.9 kg | 50 mg (2 × 25 mg capsules) | 100 mg (2 × 50 mg capsules) | 200 mg (2 × 100 mg capsules) | 6.9 | 2 capsules |
| 50 to 69.9 kg | 75 mg (3 × 25 mg capsules) | 150 mg (3 × 50 mg capsules) | 300 mg (3 × 100 mg capsules) | 7.0 | 3 capsules |
| > 70 kg | 100 mg (4 × 25 mg capsules) | 200 mg (4 × 50 mg capsules) | 400 mg (4 × 100 mg capsules) | 7.3 | 4 capsules |

| | | | | | |
|---|---|---|---|---|---|
| Abbreviations: AUC₀₋₈ = area under the curve from time 0 to 8 hours; d = day; t.i.d = 3-times daily. | | | | | |

### Evaluation Efficacy

### Primary Endpoint

The primary efficacy endpoint of the trial was the percentage change in serum chitotriosidase activity (marker of macrophage activation) from baseline to 6 months, where baseline was defined as the mean value of the predose visits (Visit 1 and Visit 2).

### Secondary Endpoints

### Growth and Maturation Measures

For patients < 18 years:
- Change in weight/length curves at 6 months and bi-annually until trial end.
- Age at pubertal onset (Tanner Stage II) for patients who had not reached puberty at screening.
- Tanner staging at baseline and 6 months, and then at least every 12 months until trial end (Tanner stage I to IV).

For adult patients ≥ 18 years, the clinical efficacy outcome measures were:
- Change from baseline in weight at 6 months and then at least every 12 months until trial end.

### Imaging Endpoint

The imaging efficacy endpoint consisted of the change in the size (centimetres) of the liver and spleen (assessed by ultrasound) at 6 months and bi-annually until trial end.

Clinical GD severity endpoints (all patients):
- Gaucher disease Type 1 Disease Severity Scoring System (GD1-DS3)
- Modified Severity Scoring Tool (mSST)

### Exploratory Endpoints

Other exploratory endpoints included blood and CSF biomarker analyses, as well as patient acceptability/palatability of IMP.

The exploratory biomarker endpoints, as measured in the blood, included:
- Change* in plasma glucosylsphingosine (GlcSph) levels at 6 months and every 6 months until trial end.
- Change* in haemoglobin, ferritin, and platelet levels at 6 months and every 6 months until trial end.
- Change* in GBA enzyme activity (leukocytes) at 6 months and every 6 months until trial end.
- Change* in serum macrophage inflammatory protein-1b (MIP-1β; cytokine for bone involvement) at 6 months and every 6 months until trial end.

*Change from baseline, baseline being defined as the mean value of the predose visits (Visit 1 and Visit 2).

The exploratory biomarker endpoints, as measured in the CSF, included:
- Change** in glycoprotein non-metastatic B at 6 months and 12 months.
- Change** in GlcSph levels at 6 months and 12 months.
- Change** in chitotriosidase activity (marker of macrophage activation) at 6 months and 12 months.
- Change** in GBA enzyme activity levels at 6 months and 12 months.
- Change** in heat shock protein 70 levels at 6 months and12 months.

**Change from Visit 2 to 6 months or 12 months after Visit 2.

### Pharmacokinetics

Blood samples after the first dose (Visit 2) were collected at the following timepoints: 0.5, 1, 2, 4, and 8 hours postdose. The AUC0-8 after the first dose was used to assess if the exposure level was acceptable. Additional blood samples for quantitative population PK analysis of arimoclomol plasma concentrations were collected predose and 0.5 hours postdose at Visit 5, and predose and 1.5 hours postdose, at Visit 7.

Cerebrospinal fluid samples for quantitative population PK analysis of arimoclomol CSF concentrations were collected predose at Visit 5, and postdose following the first daily dose at Visit 7.

### Statistical Methods

The primary efficacy endpoint was analysed using an Analysis of Covariance (ANCOVA) model on the ITT population. The log of chitotriosidase activity was used in the analysis. The response variable for ANCOVA model was the log of chitotriosidase activity at 6 months, and the independent variables included IMP dose as randomized (300 mg/day, 600 mg/day, 1200 mg/day, and 0 mg/day [for placebo]) as a continuous variable, GD type, and log of baseline chitotriosidase. Supportive analysis was conducted using the same model but with the dose actually received at Visit 3 following possible dosing adjustment based on PK assessment at Visit 2.

The ANCOVA models below were used to correlate the log of drug concentration in blood and CSF with chitotriosidase activity at 6 months in the blood/CSF population.
- ANCOVA model for log of chitotriosidase activity with log of drug concentration in CSF as measured at Visit 5, GD type and log of baseline chitotriosidase as covariates
- ANCOVA model for log of chitotriosidase activity with log of drug concentration in blood as measured at Visit 5, GD type and log of baseline chitotriosidase as covariates

Secondary efficacy endpoints, including change in weight and height, change in liver size, change in spleen size, and change in biomarkers (blood and CSF) relative to baseline (where appropriate) were analysed using an ANCOVA model with treatment group (dose actually received at Visit 3 following possible dosing adjustment based on PK assessment at Visit 2, or placebo) and corresponding baseline value as covariate. For the biomarker analysis, appropriate data transformation was conducted before statistical analysis, if necessary.

Descriptive statistics were provided by treatment group for age at pubertal onset and Tanner staging. No inferential analysis was conducted.

Safety evaluations included the following endpoints: incidence and severity of AEs (or treatment-emergent AEs [TEAEs]); C-SSRS; and mean values, change from baseline, and potentially clinically significant clinical safety laboratory tests and vital signs data. No formal statistical analysis of the safety data was performed.

Arimoclomol plasma and CSF concentrations were summarised using descriptive statistics and were summarised by Visit and time-point. The AUC0-8 after the first dose (Visit 2) was assessed using non-compartmental methods. The estimated AUC0-8 for each patient was listed.

The relationship between arimoclomol plasma and CSF concentrations with biomarkers was explored. All plasma and CSF arimoclomol concentration and dosing data were merged with the PK sampling dates and times and used to create the population PK input file for use in a population PK modelling analysis. In addition, the relationship between arimoclomol PK (plasma/CSF) and biomarkers was also explored using a population PK/PD modelling approach.

### Duration of Treatment

The duration of the proof-of-concept phase of the trial was 6 months. The open-label extension phase will continue until arimoclomol has received marketing authorisation (MA) in India or until the analysis of data from the proof-of-concept period of the present trial or information from the development program, whichever occurs first, no longer warrant its continuation.

### Duration of Patient Participation in Trial

Randomisation (Visit 2) took place 7 to 35 days after screening (Visit 1). During the proof-of-concept, double-blinded, placebo-controlled period of the trial the patient attended a visit 1 month (Visit 3), 3 months (Visit 4), and 6 months (Visit 5 [end of blinded phase]) after Visit 2. During the open-label extension phase of the trial, the patients attended a trial visit 1 month (Visit 6), 3 months (Visit 6.1) after Visit 5, 6 months (Visit 7) after Visit 5 and then every 6 months until the end of the trial. The total duration of a patient participation in the trial will depend on the date at which the MA is granted in India, or availability of relevant data from the development program.

### Results

### Treatment groups and key baseline characteristics

39 subjects were randomized in the intent-to-treat (ITT) population, however 2 patients excluded from ITT set due to negative confirmatory GD genotype analysis (post hoc). In the ITT analysis set, 31 children were children and 6 were adults.

The number of patients in each treatment group and their baseline characteristics are summarised in Table 3, below.

**Table 3 - Summary of treatment groups and key baseline characteristics.**

| **Intent-to-treat** | **Placebo** | **100 mg t.i.d.** | **200 mg t.i.d.** | **400 mg t.i.d.** | **Total** |
|---|---|---|---|---|---|
| **n** | 9 | 10 | 8 | 10 | 37 |
| **Mean age** | 10.3 | 7.8 | 11.3 | 17.4 | 11.8 |
| **Gaucher disease type 1** | 4 | 5 | 6 | 6 | 21 (56.8%) |
| **Gaucher disease type 3** | 5 | 5 | 2 | 4 | 16 (43.2%) |

### Dose-dependent plasma exposure of arimoclomol

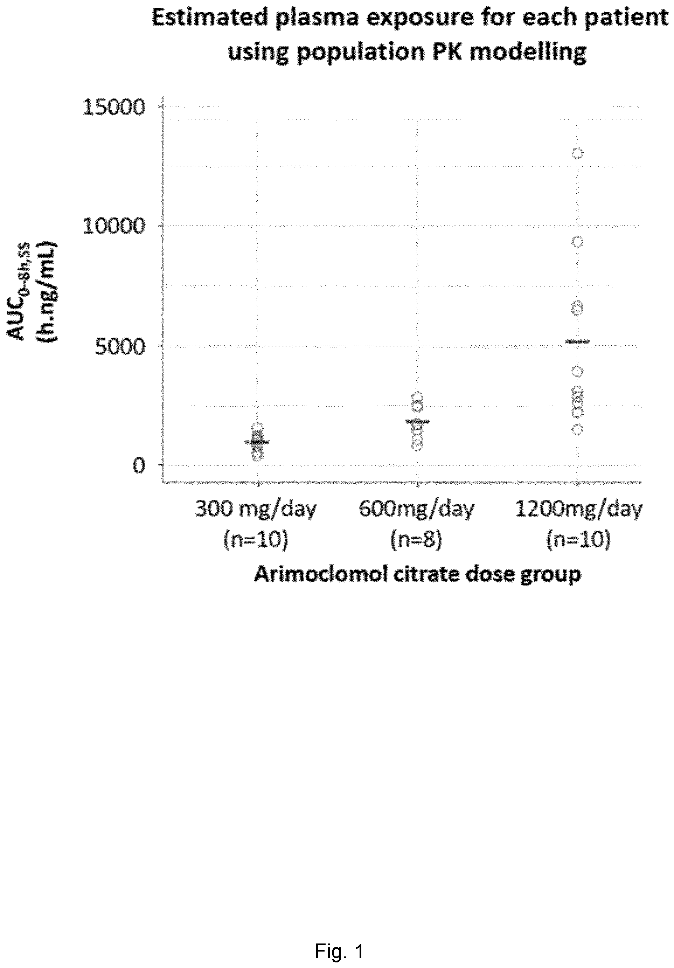

Plasma exposure (AUC) was estimated for each patient using population PK modelling. A dose-dependent increase in plasma exposure was observed after administration of arimoclomol (see Fig. 1).

### Clinical findings

### Primary endpoint

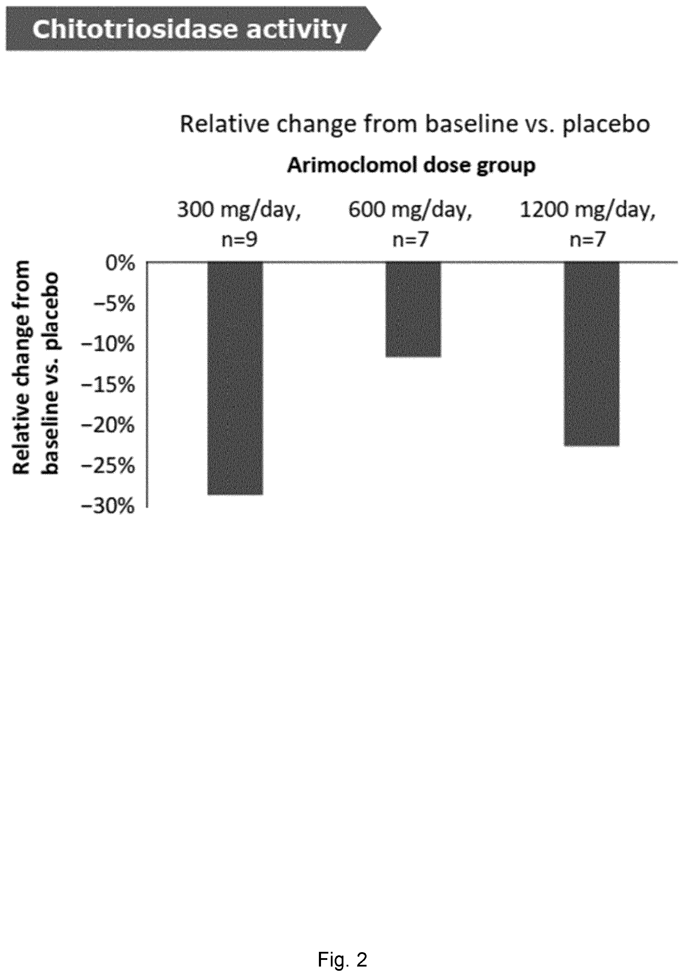

Chitotriosidase activity increased across all treatment groups, with a lower increase in arimoclomol groups vs. placebo. A relative reduction in chitotriosidase activity from baseline was observed in all dosage groups compared with placebo, though significance was not achieved (p=0.4) (see Figure 2).

### Secondary clinical endpoints

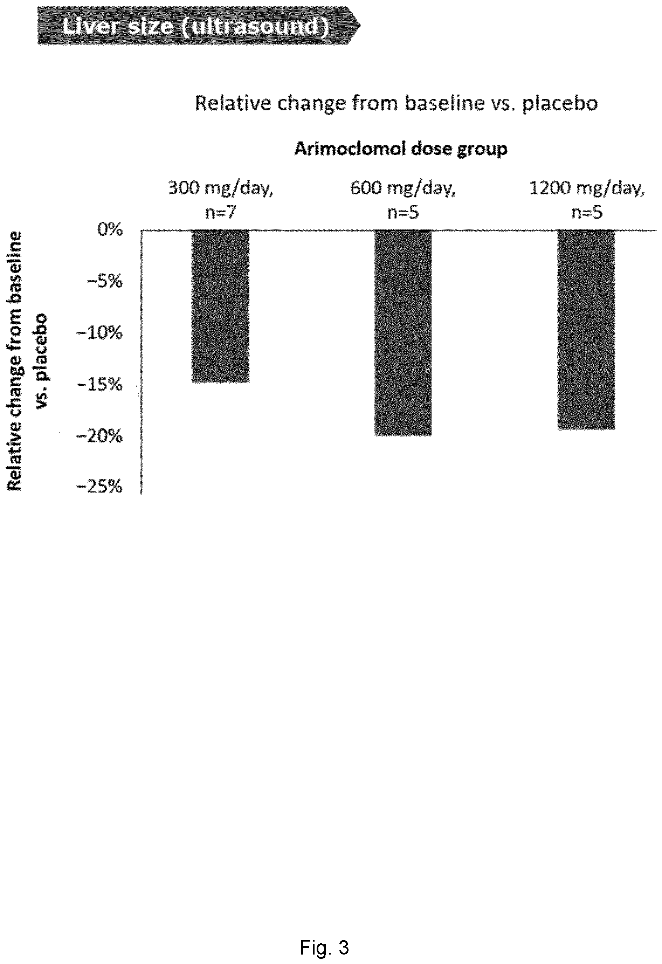

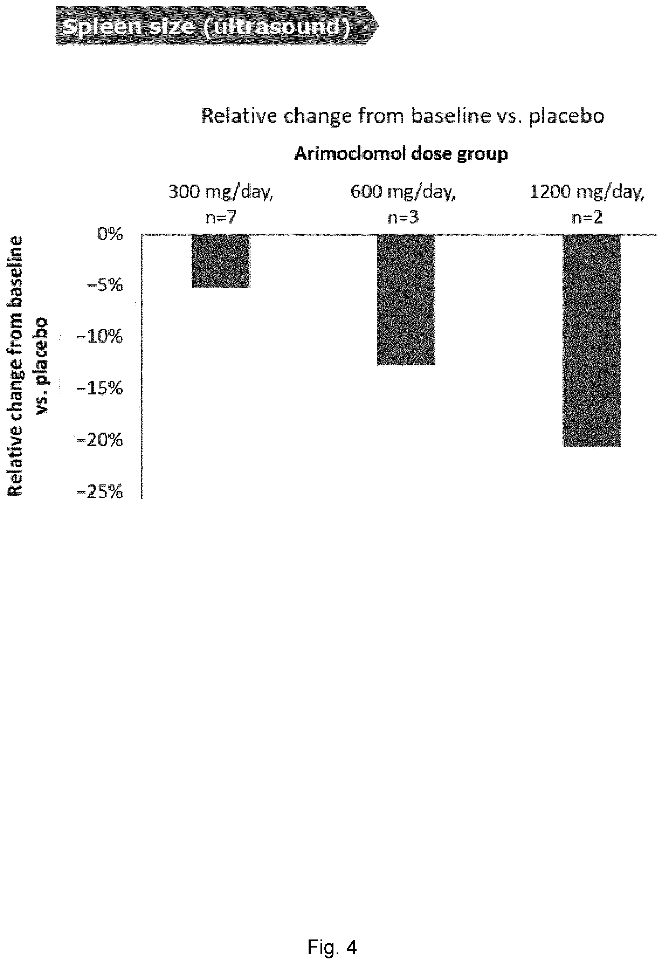

Clinically meaningful dose-dependent reductions in liver size (dose-trend effect p<0.05) and spleen size (dose trend analysis p<0.10) relative to placebo were observed (see Figures 3 and 4). A strong correlation between liver and spleen size, and treatment (correlation coefficient: 0.53) supports the consistency of effect.

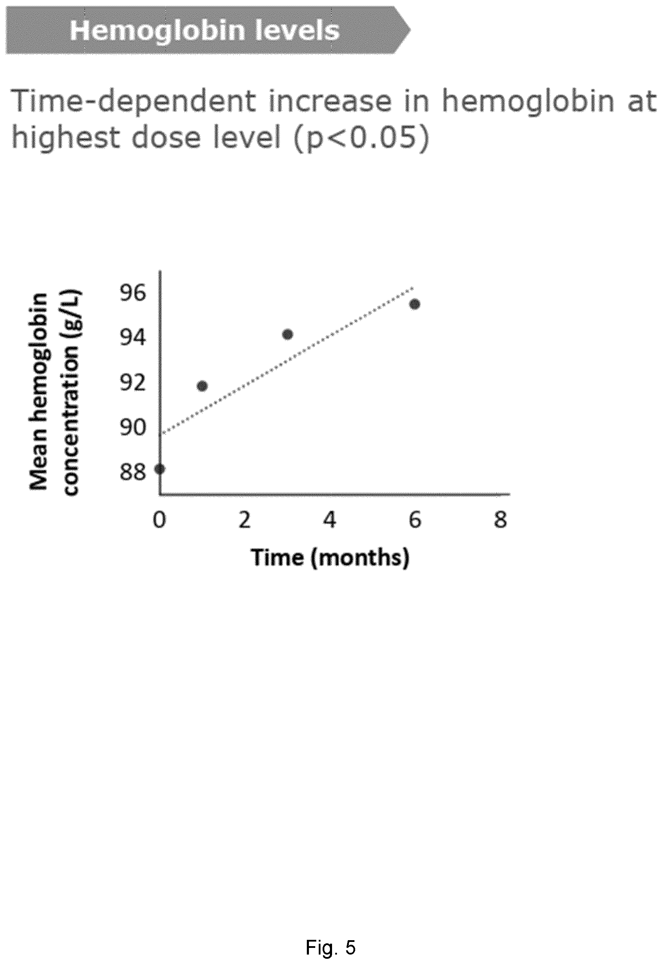

Patients anemic at baseline in higher dose group showed a time-dependent increase in haemoglobin (time-trend analysis p<0.05) (see Figure 5).

A directional dose-dependent reduction in the validated clinical composite measure of Gaucher's disease type 1 (the "Clinical GD1 severity score") was also observed, however statistical significance was not achieved.

### Other exploratory biomarkers:

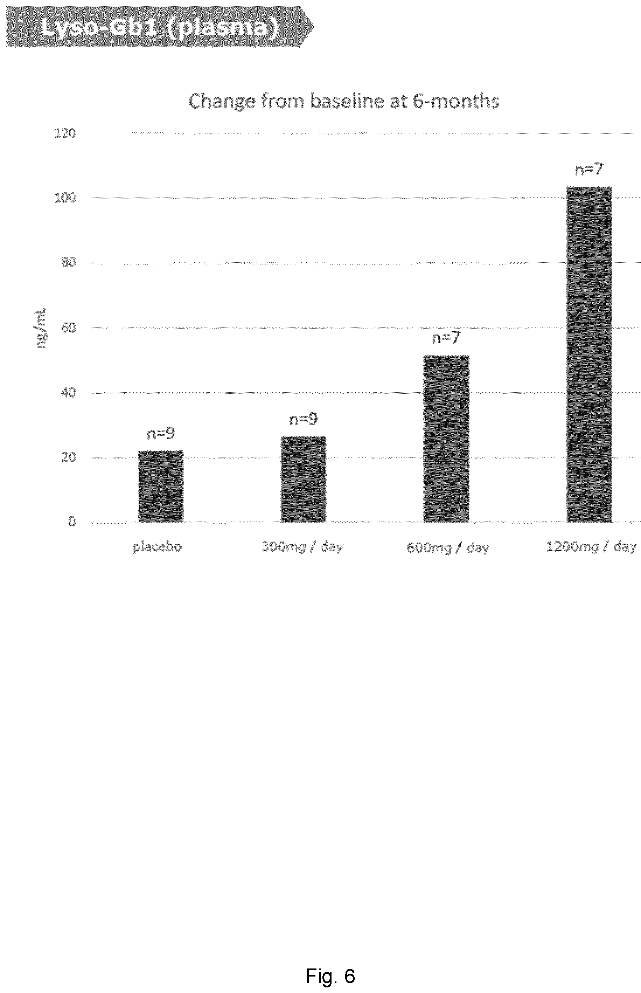

A trend towards a dose-dependent increase in glycosylsphingosine (lyso-Gb1) was observed (see Figure 6), however statistical significance was not achieved.

## Claims

1. An active pharmaceutical ingredient selected from N-[2-hydroxy-3-(1-piperidinyl)-propoxy]-pyridine-1-oxide-3-carboximidoyl chloride, its stereoisomers and the acid addition salts thereof, for use in a method of treating Gaucher disease (GD), wherein said GD is GD type 1 (GD1) or GD type 3 (GD3);
wherein said method is for use in treating hepatomegaly and/or reducing liver size in a patient with Gaucher disease,
wherein said method is for use in treating splenomegaly and/or reducing spleen size in a patient with Gaucher disease,
wherein said method is for use in treating hepatosplenomegaly and/or reducing liver size and reducing spleen size in a patient with Gaucher disease,
wherein said method is for use in treating anaemia in a patient with Gaucher disease,
wherein said method is for use in increasing haemoglobin in a patient with Gaucher disease,
wherein said method is for use in reducing serum chitotriosidase activity in a patient with Gaucher disease, and/or
wherein said method is for use in increasing glycosylsphingosine (lyso-Gb1) in a patient with Gaucher disease.

2. The active pharmaceutical ingredient for use according to claim 1, wherein said Gaucher disease is GD1 or GD3 with signs of brain involvement; such as wherein said Gaucher disease is GD3 with at least one neurological symptom.

3. The active pharmaceutical ingredient for use according to any of the preceding claims, wherein said splenomegaly is splenomegaly with anaemia and thrombocytopenia.

4. The active pharmaceutical ingredient for use according to any of the preceding claims, wherein said hepatosplenomegaly is hepatosplenomegaly with anaemia and thrombocytopenia.

5. The active pharmaceutical ingredient for use according to any of the preceding claims, wherein said patient with Gaucher disease is anaemic.

6. The active pharmaceutical ingredient for use according to any of the preceding claims, wherein said method for use in increasing haemoglobin comprises administering said active pharmaceutical ingredient at a dosage of 600 mg/day (200 mg t.i.d.) or above; such as at a dosage of 600 - 900 mg/day (200-300 mg t.i.d.) or 900-1200 mg/day (300-400 mg t.i.d.); such as wherein said method for increasing haemoglobin comprises administering said active pharmaceutical ingredient at a dosage of 600 mg/day (200 mg t.i.d.) or 1200 mg/day (400 mg t.i.d.); wherein said dosages are indicated as the citrate salt.

7. The active pharmaceutical ingredient for use according to any of the preceding claims, wherein said treatment dose-dependently reduces liver size; and/or wherein said treatment dose-dependently reduces spleen size; and/or wherein said treatment dose-dependently reduces chitotriosidase activity; and/or wherein said treatment time-dependently increases haemoglobin.

8. The active pharmaceutical ingredient for use according to any of the preceding claims, wherein the individual has not been treated for Gaucher disease within the preceding 4 months, such as with enzyme replacement therapy and/or substrate replacement therapy; or wherein the individual to be treated has not previously been treated for Gaucher disease, such as with enzyme replacement therapy and/or substrate replacement therapy; such as wherein the individual to be treated is naïve to treatment with enzyme replacement therapy and/or substrate replacement therapy.

9. The active pharmaceutical ingredient for use according to any of the preceding claims, wherein said active pharmaceutical ingredient is selected from the group consisting of:
the racemate of N-[2-hydroxy-3-(1-piperidinyl)-propoxy]-pyridine-1-oxide-3-carboximidoyl chloride,
an optically active stereoisomer of N-[2-hydroxy-3-(1-piperidinyl)-propoxy]-pyridine-1-oxide-3-carboximidoyl chloride,
an enantiomer of *N*-[2-hydroxy-3-(1-piperidinyl)-propoxy]-pyridine-1-oxide-3-carboximidoyl chloride,
(+)-(*R*)-*N*-[2-hydroxy-3-(1-piperidinyl)-propoxy]-pyridine-1-oxide-3-carboximidoyl chloride,
(-)-(*S*)-*N*-[2-hydroxy-3-(1-piperidinyl)-propoxy]-pyridine-1-oxide-3-carboximidoyl chloride,
(*Z*)-(*R*)-*N*-[2-hydroxy-3-(1-piperidinyl)-propoxy]-pyridine-1-oxide-3-carboximidoyl chloride,
(*E*)-(*R*)-*N*-[2-hydroxy-3-(1-piperidinyl)-propoxy]-pyridine-1-oxide-3-carboximidoyl chloride,
(*Z*)-(*S*)-*N*-[2-hydroxy-3-(1-piperidinyl)-propoxy]-pyridine-1-oxide-3-carboximidoyl chloride;
(*E*)-(*S*)-N-[2-hydroxy-3-(1-piperidinyl)-propoxy]-pyridine-1-oxide-3-carboximidoyl chloride,
an acid addition salt of *N*-[2-hydroxy-3-(1-piperidinyl)-propoxy]-pyridine-1-oxide-3-carboximidoyl chloride,
*N*-[2-hydroxy-3-(1-piperidinyl)-propoxy]-pyridine-1-oxide-3-carboximidoyl chloride citrate,
*N*-[2-hydroxy-3-(1-piperidinyl)-propoxy]-pyridine-1-oxide-3-carboximidoyl chloride maleate,
(+)-(*R*)-*N*-[2-hydroxy-3-(1-piperidinyl)-propoxy]-pyridine-1-oxide-3-carboximidoyl chloride citrate;
(-)-(*S*)-*N*-[2-hydroxy-3-(1-piperidinyl)-propoxy]-pyridine-1-oxide-3-carboximidoyl chloride citrate;
(+)-(*R*)-*N*-[2-hydroxy-3-(1-piperidinyl)-propoxy]-pyridine-1-oxide-3-carboximidoyl chloride maleate;
(-)-(*S*)-*N*-[2-hydroxy-3-(1-piperidinyl)-propoxy]-pyridine-1-oxide-3-carboximidoyl chloride maleate,
(*Z*)-(*R*)-*N*-[2-hydroxy-3-(1-piperidinyl)-propoxy]-pyridine-1-oxide-3-carboximidoyl chloride citrate;
(*E*)-(*R*)-*N*-[2-hydroxy-3-(1-piperidinyl)-propoxy]-pyridine-1-oxide-3-carboximidoyl chloride citrate;
(*Z*)-(*S*)-*N*-[2-hydroxy-3-(1-piperidinyl)-propoxy]-pyridine-1-oxide-3-carboximidoyl chloride citrate;
(*E*)-(*S*)-*N*-[2-hydroxy-3-(1-piperidinyl)-propoxy]-pyridine-1-oxide-3-carboximidoyl chloride citrate;
(*Z*)-(*R*)-*N*-[2-hydroxy-3-(1-piperidinyl)-propoxy]-pyridine-1-oxide-3-carboximidoyl chloride maleate;
(*E*)-(*R*)-*N*-[2-hydroxy-3-(1-piperidinyl)-propoxy]-pyridine-1-oxide-3-carboximidoyl chloride maleate;
(*Z*)-(*S*)-*N*-[2-hydroxy-3-(1-piperidinyl)-propoxy]-pyridine-1-oxide-3-carboximidoyl chloride maleate; and
(*E*)-(*S*)-*N*-[2-hydroxy-3-(1-piperidinyl)-propoxy]-pyridine-1-oxide-3-carboximidoyl chloride maleate.

10. The active pharmaceutical ingredient for use according to any of the preceding claims, wherein said active pharmaceutical ingredient is selected from the group consisting of
(+)-(*R*)-*N*-[2-hydroxy-3-(1-piperidinyl)-propoxy]-pyridine-1-oxide-3-carboximidoyl chloride (arimoclomol free base); and
(+)-(*R*)-*N*-[2-hydroxy-3-(1-piperidinyl)-propoxy]-pyridine-1-oxide-3-carboximidoyl chloride citrate (arimoclomol citrate).

11. The active pharmaceutical ingredient for use according to any one of the preceding claims, wherein the active pharmaceutical ingredient is administered daily, such as once daily, such as twice daily, such as three times daily; preferably wherein the active pharmaceutical ingredient is administered three times daily (t.i.d).

12. The active pharmaceutical ingredient for use according to any one of the preceding claims, wherein the active pharmaceutical ingredient is administered at a per administration dose of about 100 mg to about 400 mg, such as about 100 mg, 200 mg or 400 mg;
such as wherein the active pharmaceutical ingredient is administered at 100 mg t.i.d., such as 200 mg t.i.d., such as 400 mg t.i.d;
such as wherein the active pharmaceutical ingredient is administered at a daily dosage of 300 mg/day, of 600 mg/day, or of 1200 mg/day;
wherein said dosages are indicated as the citrate salt.

13. The active pharmaceutical ingredient for use according to any one of the preceding claims, wherein the active pharmaceutical ingredient is administered at a daily dosage of about 100 mg/day to about 200 mg/day, such as about 200 mg/day to about 300 mg/day, such as about 300 mg/day to about 400 mg/day, such as about 400 mg/day to about 500 mg/day, such as about 500 mg/day to about 600 mg/day, such as about 700 mg/day to about 800 mg/day, such as about 800 mg/day to about 900 mg/day, such as about 900 mg/day to about 1000 mg/day, such as about 1000 mg/day to about 1100 mg/day, such as about 1100 mg/day to about 1200 mg/day, wherein said dosages are indicated as the citrate salt.

14. The active pharmaceutical ingredient for use according to any of the preceding claims, wherein the active pharmaceutical ingredient is administered to a patient with Gaucher disease in weight-adjusted dosages or age-adjusted dosages;
such as wherein the active pharmaceutical ingredient is administered to a patient with Gaucher disease having a body weight of 10 kg to ≤ 30 kg at a dosage of 25 mg t.i.d., 50 mg t.i.d. or 100 mg t.i.d. corresponding to a dosage of 75 mg/day, 150 mg/day or 300 mg/day;
such as wherein the active pharmaceutical ingredient is administered to a patient with Gaucher disease having a body weight of 30 kg to ≤ 50 kg at a dosage of 50 mg t.i.d., 100 mg t.i.d or 200 mg t.i.d, corresponding to a dosage of 150 mg/day, 300 mg/day or 600 mg/day;
such as wherein the active pharmaceutical ingredient is administered to a patient with Gaucher disease having a body weight of 50 kg to ≤ 70 kg at a dosage of 75 mg t.i.d., 150 mg t.i.d. or 300 mg t.i.d., corresponding to a dosage of 225 mg/day, 450 mg/day, or 900 mg/day;
such as wherein the active pharmaceutical ingredient is administered to a patient with Gaucher disease having a body weight of ≥ 70 kg at a dosage of 100 mg t.i.d., 200 mg t.i.d. or 400 mg t.i.d., corresponding to a dosage of 300 mg/day, 600 mg/day or 1200 mg/day;
such as wherein the active pharmaceutical ingredient is administered to a patient with Gaucher disease aged >18 years at a dosage of 100 mg t.i.d., 200 mg t.i.d. or 400 mg t.i.d., corresponding to a dosage of 300 mg/day, 600 mg/day or 1200 mg/day;
wherein said dosages are indicated as the citrate salt.

15. The active pharmaceutical ingredient for use according to any of the preceding claims, wherein said patient with Gaucher disease is of age ≥ 4 and ≤ 60 years; such as wherein said patient with Gaucher disease is of age 4 to 18 years; and/or wherein said patient with Gaucher disease has a body weight of ≥ 10 kg.

## Patentansprüche

1. Pharmazeutischer Wirkstoff, ausgewählt aus N-[2-hydroxy-3-(1-piperidinyl)-propoxy]-pyridin-1-oxid-3-carboximidoyl-Chlorid, seinen Stereoisomeren und den Säureadditionssalzen davon, zur Verwendung in einem Verfahren zum Behandeln von Morbus Gaucher (GD), wobei der GD GD Typ 1 (GD1) oder GD Typ 3 (GD3) ist;
wobei das Verfahren zur Verwendung beim Behandeln von Hepatomegalie und/oder Verringern der Lebergröße bei einem Patienten mit Morbus Gaucher ist,
wobei das Verfahren zur Verwendung beim Behandeln von Splenomegalie und/oder Verringern der Milzgröße bei einem Patienten mit Morbus Gaucher ist,
wobei das Verfahren zur Verwendung beim Behandeln von Hepatosplenomegalie und/oder Verringern der Lebergröße und Verringern der Milzgröße bei einem Patienten mit Morbus Gaucher ist,
wobei das Verfahren zur Verwendung beim Behandeln von Anämien bei einem Patienten mit Morbus Gaucher ist,
wobei das Verfahren zur Verwendung zum Erhöhen von Hämoglobin bei einem Patienten mit Morbus Gaucher ist,
wobei das Verfahren zur Verwendung beim Verringern der Serum-Chitotriosidase-Aktivität bei einem Patienten mit Morbus Gaucher ist und/oder
wobei das Verfahren zur Verwendung beim Erhöhen von Glycosylsphingosin (Lyso-Gb1) bei einem Patienten mit Morbus Gaucher ist.

2. Pharmazeutischer Wirkstoff zur Verwendung nach Anspruch 1, wobei der Morbus Gaucher GD1 oder GD3 mit Anzeichen von Gehirnbeteiligung ist; wie etwa wobei der Morbus Gaucher GD3 mit mindestens einem neurologischen Symptom ist.

3. Pharmazeutischer Wirkstoff zur Verwendung nach einem der vorhergehenden Ansprüche, wobei die Splenomegalie eine Splenomegalie mit Anämie und Thrombozytopenie ist.

4. Pharmazeutischer Wirkstoff zur Verwendung nach einem der vorhergehenden Ansprüche, wobei die Hepatosplenomegalie eine Hepatosplenomegalie mit Anämie und Thrombozytopenie ist.

5. Pharmazeutischer Wirkstoff zur Verwendung nach einem der vorhergehenden Ansprüche, wobei der Patient mit Morbus Gaucher anämisch ist.

6. Pharmazeutischer Wirkstoff zur Verwendung nach einem der vorhergehenden Ansprüche, wobei das Verfahren zur Verwendung beim Erhöhen des Hämoglobins ein Verabreichen des pharmazeutischen Wirkstoffs in einer Dosierung von 600 mg/Tag (200 mg t.i.d.) oder höher umfasst; wie etwa in einer Dosierung von 600-900 mg/Tag (200-300 mg t.i.d.) oder 900-1200 mg/Tag (300-400 mg t.i.d.); wie etwa wobei das Verfahren zum Erhöhen des Hämoglobins ein Verabreichen des pharmazeutischen Wirkstoffs in einer Dosierung von 600 mg/Tag (200 mg t.i.d.) oder 1200 mg/Tag (400 mg t.i.d.) umfasst; wobei die Dosierungen als das Zitratsalz angegeben sind.

7. Pharmazeutischer Wirkstoff zur Verwendung nach einem der vorhergehenden Ansprüche, wobei die Behandlung dosisabhängig die Lebergröße reduziert; und/oder wobei die Behandlung dosisabhängig die Milzgröße reduziert; und/oder wobei die Behandlung dosisabhängig die Chitotriosidase-Aktivität reduziert; und/oder wobei die Behandlung zeitabhängig das Hämoglobin erhöht.

8. Pharmazeutischer Wirkstoff zur Verwendung nach einem der vorangehenden Ansprüche, wobei die Person innerhalb der vorangehenden 4 Monate nicht wegen Morbus Gaucher behandelt wurde, wie etwa mit einer Enzymersatztherapie und/oder Substratersatztherapie; oder wobei die zu behandelnde Person zuvor nicht wegen Morbus Gaucher behandelt wurde, wie etwa mit einer Enzymersatztherapie und/oder Substratersatztherapie; wie etwa wobei die zu behandelnde Person behandlungsnaiv mit einer Enzymersatztherapie und/oder Substratersatztherapie ist.

9. Pharmazeutischer Wirkstoff zur Verwendung nach einem der vorhergehenden Ansprüche, wobei der pharmazeutische Wirkstoff ausgewählt ist aus der Gruppe, bestehend aus:
dem Racemat von *N*-[2-hydroxy-3-(1-piperidinyl)-propoxy]-pyridin-1-oxid-3-carboximidoyl-Chlorid,
einem optisch aktiven Stereoisomer von *N*-[2-hydroxy-3-(1-piperidinyl)-propoxy]-pyridin-1-oxid-3-carboximidoyl-Chlorid,
einem Enantiomer von *N*-[2-hydroxy-3-(1-piperidinyl)-propoxy]-pyridin-1-oxid-3-carboximidoyl-Chlorid,
(+)-(*R*)-*N*-[2-hydroxy-3-(1-piperidinyl)-propoxy]-pyridin-1-oxid-3-carboximidoyl-Chlorid,
(-)-(*S*)-*N*-[2-hydroxy-3-(1-piperidinyl)-propoxy]-pyridin-1-oxid-3-carboximidoyl-Chlorid,
(*Z*)-(*R*)-*N*-[2-hydroxy-3-(1-piperidinyl)-propoxy]-pyridin-1-oxid-3-carboximidoyl-Chlorid,
(*E*)-(*R*)-*N*-[2-hydroxy-3-(1-piperidinyl)-propoxy]-pyridin-1-oxid-3-carboximidoyl-Chlorid,
(*Z*)-(*S*)-*N*-[2-hydroxy-3-(1-piperidinyl)-propoxy]-pyridin-1-oxid-3-carboximidoyl-Chlorid,
(*E*)-(*S*)-*N*-[2-hydroxy-3-(1-piperidinyl)-propoxy]-pyridin-1-oxid-3-carboximidoyl-Chlorid,
einem Säureadditionssalz von *N*-[2-hydroxy-3-(1-piperidinyl)-propoxy]-pyridin-1-oxid-3-carboximidoyl-Chlorid,
*N*-[2-hydroxy-3-(1-piperidinyl)-propoxy]-pyridin-1-oxid-3-carboximidoyl-Chloridzitrat,
*N*-[2-hydroxy-3-(1-piperidinyl)-propoxy]-pyridin-1-oxid-3-carboximidoyl-Chloridmaleat,
(+)-(*R*)-*N*-[2-hydroxy-3-(1-piperidinyl)-propoxy]-pyridin-1-oxid-3-carboximidoyl-Chloridzitrat;
(-)-(*S*)-*N*-[2-hydroxy-3-(1-piperidinyl)-propoxy]-pyridin-1-oxid-3-carboximidoyl-Chloridzitrat,
(+)-(*R*)-*N*-[2-hydroxy-3-(1-piperidinyl)-propoxy]-pyridin-1-oxid-3-carboximidoyl-Chloridmaleat;
(-)-(*S*)-*N*-[2-hydroxy-3-(1-piperidinyl)-propoxy]-pyridin-1-oxid-3-carboximidoyl-Chloridmaleat,
(*Z*)-*R*-*N*-[2-hydroxy-3-(1-piperidinyl)-propoxy]-pyridin-1-oxid-3-carboximidoyl-Chloridzitrat;
(*E*)-(*R*)-*N*-[2-hydroxy-3-(1-piperidinyl)-propoxy]-pyridin-1-oxid-3-carboximidoyl-Chloridzitrat;
(*Z*)-(*S*)-*N*-[2-hydroxy-3-(1-piperidinyl)-propoxy]-pyridin-1-oxid-3-carboximidoyl-Chloridzitrat,
(*E*)-(*S*)-*N*-[2-hydroxy-3-(1-piperidinyl)-propoxy]-pyridin-1-oxid-3-carboximidoyl-Chloridzitrat,
(*Z*)-(*R*)-*N*-[2-hydroxy-3-(1-piperidinyl)-propoxy]-pyridin-1-oxid-3-carboximidoyl-Chloridmaleat;
(*E*)-(*R*)-*N*-[2-hydroxy-3-(1-piperidinyl)-propoxy]-pyridin-1-oxid-3-carboximidoyl-Chloridmaleat;
(*Z*)-(*S*)-*N*-[2-hydroxy-3-(1-piperidinyl)-propoxy]-pyridin-1-oxid-3-carboximidoyl-Chloridmaleat; und
(*E*)-(*S*)-*N*-[2-hydroxy-3-(1-piperidinyl)-propoxy]-pyridin-1-oxid-3-carboximidoyl-Chloridmaleat;

10. Pharmazeutischer Wirkstoff zur Verwendung nach einem der vorhergehenden Ansprüche, wobei der pharmazeutische Wirkstoff ausgewählt ist aus der Gruppe, bestehend aus
(+)-(*R*-)-*N*-[2-hydroxy-3-(1-piperidinyl)-propoxy]-pyridin-1-oxid-3-carboximidoyl-Chlorid (freie Base von Arimoclomol); und
(+)-(*R*)-*N*-[2-hydroxy-3-(1-piperidinyl)-propoxy]-pyridin-1-oxid-3-carboximidoyl-Chloridzitrat (Arimoclomolzitrat).

11. Pharmazeutischer Wirkstoff zur Verwendung nach einem der vorhergehenden Ansprüche, wobei der pharmazeutische Wirkstoff täglich, wie etwa einmal täglich, wie etwa zweimal täglich, wie etwa dreimal täglich verabreicht wird; wobei der pharmazeutische Wirkstoff vorzugsweise dreimal täglich (t.i.d.) verabreicht wird.

12. Pharmazeutischer Wirkstoff zur Verwendung nach einem der vorhergehenden Ansprüche, wobei der pharmazeutische Wirkstoff pro Verabreichung in einer Dosis von ca. 100 mg bis ca. 400 mg, wie etwa ca. 100 mg, 200 mg oder 400 mg, verabreicht wird;
wie etwa wobei der pharmazeutische Wirkstoff in einer Dosierung von 100 mg t.i.d., wie etwa 200 mg t.i.d., wie etwa 400 mg t.i.d., verabreicht wird;
wie etwa wobei der pharmazeutische Wirkstoff in einer Tagesdosis von 300 mg/Tag, von 600 mg/Tag oder von 1200 mg/Tag verabreicht wird;
wobei die Dosierungen als das Zitratsalz angegeben sind.

13. Pharmazeutischer Wirkstoff zur Verwendung nach einem der vorhergehenden Ansprüche, wobei der pharmazeutische Wirkstoff in einer Tagesdosis von ca. 100 mg/Tag bis ca. 200 mg/Tag verabreicht wird, wie etwa ca. 200 mg/Tag bis ca. 300 mg/Tag, wie etwa ca. 300 mg/Tag bis ca. 400 mg/Tag, wie etwa ca. 400 mg/Tag bis ca. 500 mg/Tag, wie etwa ca. 500 mg/Tag bis ca. 600 mg/Tag, wie etwa ca. 700 mg/Tag bis ca. 800 mg/Tag, wie etwa ca. 800 mg/Tag bis ca. 900 mg/Tag, wie etwa ca. 900 mg/Tag bis ca. 1000 mg/Tag, wie etwa ca. 1000 mg/Tag bis ca. 1100 mg/Tag, wie etwa ca. 1100 mg/Tag bis ca. 1200 mg/Tag, wobei die Dosierungen als das Zitratsalz angegeben sind.

14. Pharmazeutischer Wirkstoff zur Verwendung nach einem der vorhergehenden Ansprüche, wobei der pharmazeutische Wirkstoff einem Patienten mit Morbus Gaucher in gewichtsangepassten Dosierungen oder altersangepassten Dosierungen verabreicht wird;
wie etwa wobei der pharmazeutische Wirkstoff einem Patienten mit Morbus Gaucher, der ein Körpergewicht von 10 kg bis ≤ 30 kg aufweist, in einer Dosierung von 25 mg t.i.d., 50 mg t.i.d. oder 100 mg t.i.d., entsprechend einer Dosierung von 75 mg/Tag, 150 mg/Tag oder 300 mg/Tag, verabreicht wird;
wie etwa wobei der pharmazeutische Wirkstoff einem Patienten mit Morbus Gaucher, der ein Körpergewicht von 30 kg bis ≤ 50 kg aufweist, in einer Dosierung von 50 mg t.i.d., 100 mg t.i.d. oder 200 mg t.i.d., entsprechend einer Dosierung von 150 mg/Tag, 300 mg/Tag oder 600 mg/Tag, verabreicht wird;
wie etwa wobei der pharmazeutische Wirkstoff einem Patienten mit Morbus Gaucher, der ein Körpergewicht von 50 kg bis ≤ 70 kg aufweist, in einer Dosierung von 75 mg t.i.d., 150 mg t.i.d. oder 300 mg t.i.d., entsprechend einer Dosierung von 225 mg/Tag, 450 mg/Tag oder 900 mg/Tag, verabreicht wird;
wie etwa wobei der pharmazeutische Wirkstoff einem Patienten mit Morbus Gaucher, der ein Körpergewicht von ≥ 70 kg aufweist, in einer Dosierung von 100 mg t.i.d., 200 mg t.i.d. oder 400 mg t.i.d., entsprechend einer Dosierung von 300 mg/Tag, 600 mg/Tag oder 1200 mg/Tag, verabreicht wird;
wie etwa wobei der pharmazeutische Wirkstoff einem Patienten mit Morbus Gaucher in einem Alter von > 18 Jahren in einer Dosierung von 100 mg t.i.d., 200 mg t.i.d. oder 400 mg t.i.d., entsprechend einer Dosierung von 300 mg/Tag, 600 mg/Tag oder 1200 mg/Tag, verabreicht wird;
wobei die Dosierungen als das Zitratsalz angegeben sind.

15. Pharmazeutischer Wirkstoff zur Verwendung nach einem der vorhergehenden Ansprüche, wobei der Patient mit Morbus Gaucher in einem Alter von ≥ 4 und ≤ 60 Jahren ist; wie etwa wobei der Patient mit Morbus Gaucher in einem Alter von 4 bis 18 Jahren ist; und/oder wobei der Patient mit Morbus Gaucher ein Körpergewicht von ≥ 10 kg aufweist.

## Revendications

1. Ingrédient pharmaceutique actif choisi parmi le chlorure de *N*-[2-hydroxy-3-(1-pipéridinyl)-propoxy]-pyridine-1-oxyde-3-carboximidoyle, ses stéréoisomères et les sels d'addition d'acide de celui-ci, pour une utilisation dans un procédé de traitement de la maladie de Gaucher (GD), dans lequel ladite GD est la GD de type 1 (GD1) ou la GD de type 3 (GD3) ;
dans lequel ledit procédé est pour une utilisation dans le traitement de l'hépatomégalie et/ou la réduction de la taille du foie chez un patient atteint de la maladie de Gaucher,
dans lequel ledit procédé est pour une utilisation dans le traitement de la splénomégalie et/ou la réduction de la taille de la rate chez un patient atteint de la maladie de Gaucher,
dans lequel ledit procédé est pour une utilisation dans le traitement de l'hépatosplénomégalie et/ou la réduction de la taille du foie et la réduction de la taille de la rate chez un patient atteint de la maladie de Gaucher,
dans lequel ledit procédé est pour une utilisation dans le traitement de l'anémie chez un patient atteint de la maladie de Gaucher,
dans lequel ledit procédé est pour une utilisation dans l'augmentation de l'hémoglobine chez un patient atteint de la maladie de Gaucher,
dans lequel ledit procédé est pour une utilisation dans la réduction de l'activité de la chitotriosidase sérique chez un patient atteint de la maladie de Gaucher, et/ou
dans lequel ledit procédé est pour une utilisation dans l'augmentation de la glycosylsphingosine (lyso-Gb1) chez un patient atteint de la maladie de Gaucher.

2. Ingrédient pharmaceutique actif pour une utilisation selon la revendication 1, dans lequel ladite maladie de Gaucher est la GD1 ou la GD3 avec des signes d'atteinte cérébrale ; par exemple dans lequel ladite maladie de Gaucher est la GD3 avec au moins un symptôme neurologique.

3. Ingrédient pharmaceutique actif pour une utilisation selon l'une quelconque des revendications précédentes, dans lequel ladite splénomégalie est une splénomégalie avec anémie et thrombocytopénie.

4. Ingrédient pharmaceutique actif pour une utilisation selon l'une quelconque des revendications précédentes, dans lequel ladite hépatosplénomégalie est une hépatosplénomégalie avec anémie et thrombocytopénie.

5. Ingrédient pharmaceutique actif pour une utilisation selon l'une quelconque des revendications précédentes, dans lequel ledit patient atteint de la maladie de Gaucher est anémique.

6. Ingrédient actif pharmaceutique pour une utilisation selon l'une quelconque des revendications précédentes, dans lequel ledit procédé d'utilisation pour une augmentation de l'hémoglobine comprend l'administration dudit ingrédient pharmaceutique actif à une dose de 600 mg/jour (200 mg t.i.d.) ou plus ; par exemple à une dose de 600 à 900 mg/jour (200 à 300 mg t.i.d.) ou de 900 à 1200 mg/jour (300 à 400 mg t.i.d.) ; par exemple, dans lequel ledit procédé pour une augmentation de l'hémoglobine comprend l'administration dudit ingrédient pharmaceutique actif à une dose de 600 mg/jour (200 mg t.i.d.) ou de 1200 mg/jour (400 mg t.i.d.) ; dans lequel lesdites doses sont indiquées sous la forme du sel citrate.

7. Ingrédient pharmaceutique actif pour une utilisation selon l'une quelconque des revendications précédentes, dans lequel ledit traitement réduit la taille du foie de manière dose-dépendante ; et/ou dans lequel ledit traitement réduit la taille de la rate de manière dose-dépendante ; et/ou dans lequel ledit traitement réduit l'activité de la chitotriosidase de manière dose-dépendante ; et/ou dans lequel ledit traitement augmente l'hémoglobine de manière temps-dépendante.

8. Ingrédient pharmaceutique actif pour une utilisation selon l'une quelconque des revendications précédentes, dans lequel l'individu n'a pas été traité pour la maladie de Gaucher au cours des 4 mois précédents, par exemple par une thérapie de remplacement enzymatique et/ou une thérapie de remplacement de substrat ; ou dans lequel l'individu à traiter n'a pas été traité auparavant pour la maladie de Gaucher, par exemple par une thérapie de remplacement enzymatique et/ou une thérapie de remplacement de substrat ; par exemple dans lequel l'individu à traiter est naïf de traitement par une thérapie de remplacement enzymatique et/ou une thérapie de remplacement de substrat.

9. Ingrédient pharmaceutique actif pour une utilisation selon l'une quelconque des revendications précédentes, dans lequel ledit ingrédient pharmaceutique actif est choisi dans le groupe constitué par :
le racémate du chlorure de *N*-[2-hydroxy-3-(1-pipéridinyl)-pro-poxy]-pyridine-1-oxyde-3-carboximidoyle,
un stéréoisomère optiquement actif du chlorure de *N*-[2-hydroxy-3-(1-pipéridinyl)-propoxy]-pyridine-1-oxyde-3-carboximidoyle,
un énantiomère du chlorure de *N*-[2-hydroxy-3-(1-pipéridinyl)-propoxy]-pyridine-1-oxyde-3-carboximidoyle,
le chlorure de (+)-(*R*)-*N*-[2-hydroxy-3-(1-pipéridinyl)-propoxy]-pyridine-1-oxyde-3-carboximidoyle,
le chlorure de (-)-(*S*)-*N*-[2-hydroxy-3-(1-pipéridinyl)-propoxy]-pyridine-1-oxyde-3-carboximidoyle,
le chlorure de (*Z*)-(*R*)-*N*-[2-hydroxy-3-(1-pipéridinyl)-propoxy]-pyridine-1-oxyde-3-carboximidoyle,
le chlorure de (*E*)-(*R*)-*N*-[2-hydroxy-3-(1-pipéridinyl)-propoxy]-pyridine-1-oxyde-3-carboximidoyle,
le chlorure de (*Z*)-(*S*)-*N*-[2-hydroxy-3-(1-pipéridinyl)-propoxy]-pyridine-1-oxyde-3-carboximidoyle,
le chlorure de (*E*)-(*S*)-*N*-[2-hydroxy-3-(1-pipéridinyl)-propoxy]-pyridine-1-oxyde-3-carboximidoyle,
un sel d'addition d'acide du chlorure de *N*-[2-hydroxy-3-(1-pi-péridinyl)-propoxy]-pyridine-1-oxyde-3-carboximidoyle,
le citrate de chlorure de *N*-[2-hydroxy-3-(1-pipéridinyl)-pro-poxy]-pyridine-1-oxyde-3-carboximidoyle,
le maléate de chlorure de *N*-[2-hydroxy-3-(1-pipéridinyl)-pro-poxy]-pyridine-1-oxyde-3-carboximidoyle,
le citrate de chlorure de (+)-(*R*)-*N*-[2-hydroxy-3-(1-pipéridinyl)-propoxy]-pyridine-1-oxyde-3-carboximidoyle ;
le citrate de chlorure de (-)-(*S*)-*N*-[2-hydroxy-3-(1-pipéridinyl)-propoxy]-pyridine-1-oxyde-3-carboximidoyle ;
le maléate de chlorure de (+)-(*R*)-*N*-[2-hydroxy-3-(1-pipéridinyl)-propoxy]-pyridine-1-oxyde-3-carboximidoyle ;
le maléate de chlorure de (-)-(*S*)-*N*-[2-hydroxy-3-(1-pipéridinyl)-propoxy]-pyridine-1-oxyde-3-carboximidoyle ;
le citrate de chlorure de (*Z*)-(R)-*N-*[2-hydroxy-3-(1-pipéridinyl)-propoxy]-pyridine-1-oxyde-3-carboximidoyle ;
le citrate de chlorure de (*E)*-(R)-*N*-[2-hydroxy-3-(1-pipéridinyl)-propoxy]-pyridine-1-oxyde-3-carboximidoyle ;
le citrate de chlorure de (*Z*)-(*S*)*-N*-[2-hydroxy-3-(1-pipéridinyl)-propoxy]-pyridine-1-oxyde-3-carboximidoyle ;
le citrate de chlorure de (*E*)-(*S*)-*N*-[2-hydroxy-3-(1-pipéridinyl)-propoxy]-pyridine-1-oxyde-3-carboximidoyle ;
le maléate de chlorure de (*Z*)-(*R*)-*N*-[2-hydroxy-3-(1-pipéridinyl)-propoxy]-pyridine-1-oxyde-3-carboximidoyle ;
le maléate de chlorure de (*E*)-(*R*)-*N*-[2-hydroxy-3-(1-pipéridinyl)-propoxy]-pyridine-1-oxyde-3-carboximidoyle ;
le maléate de chlorure de (*Z*)-(*S*)-*N*-[2-hydroxy-3-(1-pipéridinyl)-propoxy]-pyridine-1-oxyde-3-carboximidoyle ; et
le maléate de chlorure de (*E*)-(*S*)-*N*-[2-hydroxy-3-(1-pipéridinyl)-propoxy]-pyridine-1-oxyde-3-carboximidoyle.

10. Ingrédient pharmaceutique actif pour une utilisation selon l'une quelconque des revendications précédentes, dans lequel ledit ingrédient pharmaceutique actif est choisi dans le groupe constitué par
le chlorure de (+)-(*R*)-*N*-[2-hydroxy-3-(1-pipéridinyl)-propoxy]-pyridine-1-oxyde-3-carboximidoyle (base libre d'arimoclomol) ; et
le citrate de chlorure de (+)-(*R*)-*N*-[2-hydroxy-3-(1-pipéridinyl)-propoxy]-pyridine-1-oxyde-3-carboximidoyle (citrate d'arimoclomol).

11. Ingrédient pharmaceutique actif pour une utilisation selon l'une quelconque des revendications précédentes, dans lequel l'ingrédient pharmaceutique actif est administré quotidiennement, par exemple une fois par jour, par exemple deux fois par jour, par exemple trois fois par jour ; de préférence dans lequel l'ingrédient pharmaceutique actif est administré trois fois par jour (t.i.d.).

12. Ingrédient pharmaceutique actif pour une utilisation selon l'une quelconque des revendications précédentes, dans lequel l'ingrédient pharmaceutique actif est administré à une dose par administration d'environ 100 mg à environ 400 mg, par exemple environ 100 mg, 200 mg ou 400 mg ;
par exemple dans lequel l'ingrédient pharmaceutique actif est administré à raison de 100 mg t.i.d., par exemple de 200 mg t.i.d., par exemple de 400 mg t.i.d. ;
par exemple dans lequel l'ingrédient pharmaceutique actif est administré à une dose quotidienne de 300 mg/jour, de 600 mg/jour ou de 1200 mg/jour ;
dans lequel lesdites doses sont indiquées sous la forme du sel citrate.

13. Ingrédient pharmaceutique actif pour une utilisation selon l'une quelconque des revendications précédentes, dans lequel l'ingrédient pharmaceutique actif est administré à une dose quotidienne d'environ 100 mg/jour à environ 200 mg/jour, par exemple d'environ 200 mg/jour à environ 300 mg/jour, par exemple d'environ 300 mg/jour à environ 400 mg/jour, par exemple d'environ 400 mg/jour à environ 500 mg/jour, par exemple d'environ 500 mg/jour à environ 600 mg/jour, par exemple d'environ 700 mg/jour à environ 800 mg/jour, par exemple d'environ 800 mg/jour à environ 900 mg/jour, par exemple d'environ 900 mg/jour à environ 1000 mg/jour, par exemple d'environ 1000 mg/jour à environ 1100 mg/jour, par exemple d'environ 1100 mg/jour à environ 1200 mg/jour, dans lequel lesdites doses sont indiquées sous la forme du sel citrate.

14. Ingrédient pharmaceutique actif pour une utilisation selon l'une quelconque des revendications précédentes, dans lequel l'ingrédient pharmaceutique actif est administré à un patient atteint de la maladie de Gaucher à des doses ajustées en fonction du poids ou à des doses ajustées en fonction de l'âge ;
par exemple dans lequel l'ingrédient pharmaceutique actif est administré à un patient atteint de la maladie de Gaucher ayant un poids corporel de 10 kg à ≤ 30 kg à une dose de 25 mg t.i.d., de 50 mg t.i.d. ou de 100 mg t.i.d. correspondant à une dose de 75 mg/jour, 150 mg/jour ou 300 mg/jour ;
par exemple dans lequel l'ingrédient pharmaceutique actif est administré à un patient atteint de la maladie de Gaucher ayant un poids corporel de 30 kg à ≤ 50 kg à une dose de 50 mg t.i.d., de 100 mg t.i.d. ou de 200 mg t.i.d. correspondant à une dose de 150 mg/jour, 300 mg/jour ou 600 mg/jour ;
par exemple dans lequel l'ingrédient pharmaceutique actif est administré à un patient atteint de la maladie de Gaucher ayant un poids corporel de 50 kg à ≤ 70 kg à une dose de 75 mg t.i.d., de 150 mg t.i.d. ou de 300 mg t.i.d. correspondant à une dose de 225 mg/jour, 450 mg/jour ou 900 mg/jour ;
par exemple, dans lequel l'ingrédient pharmaceutique actif est administré à un patient atteint de la maladie de Gaucher ayant un poids corporel ≥ 70 kg, à une dose de 100 mg t.i.d., 200 mg t.i.d. ou 400 mg t.i.d. correspondant à une dose de 300 mg/jour, 600 mg/jour ou 1200 mg/jour ;
par exemple dans lequel l'ingrédient pharmaceutique actif est administré à un patient atteint de la maladie de Gaucher âgé de plus de 18 ans à une dose de 100 mg t.i.d., de 200 mg t.i.d. ou de 400 mg t.i.d. correspondant à une dose de 300 mg/jour, 600 mg/jour ou 1200 mg/jour ;
dans lequel lesdites doses sont indiquées sous la forme du sel citrate.

15. Ingrédient pharmaceutique actif pour une utilisation selon l'une quelconque des revendications précédentes, dans lequel ledit patient atteint de la maladie de Gaucher est âgé de ≥ 4 ans et ≤ 60 ans ; par exemple dans lequel ledit patient atteint de la maladie de Gaucher est âgé de 4 à 18 ans ; et/ou dans lequel ledit patient atteint de la maladie de Gaucher a un poids corporel de ≥ 10 kg.
